# EUROPEAN PATENT SPECIFICATION

(11) **EP 1 639 109 B1**
(45) Date of publication and mention of the grant of the patent: **13.04.2011**
(21) Application number: 04754961.3
(22) Date of filing: 09.06.2004
(51) Int. Cl.: C12N 15/00, C12N 15/09, C12N 5/10, C12N 15/63, C12N 15/74

(54) **DNA VECTORS**
DNA-VEKTOREN
VECTEURS ADN

(30) Priority: 09.06.2003 US 477232 P
(43) Date of publication of application: 29.03.2006
(73) Proprietor: CORIXA CORPORATION, Seattle, WA 98104 (US)
(72) Inventor: SPIES, Gregory, A., Shoreline, WA 98155 (US); MISHER, Lynda, Seattle, WA 98105 (US)
(74) Representative: O'Farrell, Damien John
(86) International application number: PCT/US2004/018529
(87) International publication number: WO 2005/002509

(56) References cited:
- US-A- 5 547 932
- US-B1- 6 534 482
- NORMAN J A ET AL: "Development of improved vectors for DNA-based immunization and other gene therapy applications" VACCINE, BUTTERWORTH SCIENTIFIC. GUILDFORD, GB, vol. 15, no. 8, June 1997 (1997-06), pages 801-803, XP004075655 ISSN: 0264-410X
- FOECKING M K ET AL: "POWERFUL AND VERSATILE ENHANCER-PROMOTER UNIT FOR MAMMALIAN EXPRESSION VECTORS" GENE, ELSEVIER, AMSTERDAM, NL, vol. 45, no. 1, 1986, pages 101-105, XP009061501 ISSN: 0378-1119
- BRIGHTWELL G ET AL: "Serum-dependent and cell cycle-dependent expression from a cytomegalovirus-based mammalian expression vector" GENE: AN INTERNATIONAL JOURNAL ON GENES AND GENOMES, ELSEVIER, AMSTERDAM, NL, vol. 194, no. 1, 18 July 1997 (1997-07-18), pages 115-123, XP004084667 ISSN: 0378-1119
- XU Z-L ET AL: "Strength evaluation of transcriptional regulatory elements for transgene expression by adenovirus vector" JOURNAL OF CONTROLLED RELEASE, ELSEVIER, AMSTERDAM, NL, vol. 81, no. 1-2, 17 May 2002 (2002-05-17), pages 155-163, XP004351113 ISSN: 0168-3659
- XU Z.-L. ET AL: 'Optimization of Transcriptional Regulatory Elements for Constructing Plasmid Vectors' GENE vol. 272, no. 1-2, July 2001, pages 149 - 156, XP002279973
- WANG T. ET AL: 'L523S, An RNA-Binding Protein as a Potential Therapeutic Target for Lung Cancer' vol. 88, no. 6, March 2003, pages 887 - 894, XP002985223
- GARMORY H.S. ET AL: 'DNA Vaccines: Improving Expression of Antigens' GENETIC VACCINES AND THERAPY, [Online] vol. 1, September 2003, page 5, XP002985224 Retrieved from the Internet: <URL:http://www.gvt-journal.com/content/1/1 /2>

### INFORMAL SEQUENCE LISTING

<210> 1
<211> 2665
<212> DNA
<213> pUC9
<400> 1
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac catgattacg ccaagcttgg 240
   ctgcaggtcg acggatcccc gggaattcac tggccgtcgt tttacaacgt cgtgactggg 300
   aaaaccctgg cgttacccaa cttaatcgcc ttgcagcaca tccccctttc gccagctggc 360
   gtaatagcga agaggcccgc accgatcgcc cttcccaaca gttgcgcagc ctgaatggcg 420
   aatggcgcct gatgcggtat tttctcctta cgcatctgtg cggtatttca caccgcatat 480
   ggtgcactct cagtacaatc tgctctgatg ccgcatagtt aagccagccc cgacacccgc 540
   caacacccgc tgacgcgccc tgacgggctt gtctgctccc ggcatccgct tacagacaag 600
   ctgtgaccgt ctccgggagc tgcatgtgtc agaggttttc accgtcatca ccgaaacgcg 660
   cgagacgaaa gggcctcgtg atacgcctat ttttataggt taatgtcatg ataataatgg 720
   tttcttagac gtcaggtggc acttttcggg gaaatgtgcg cggaacccct atttgtttat 780
   ttttctaaat acattcaaat atgtatccgc tcatgagaca ataaccctga taaatgcttc 840
   aataatattg aaaaaggaag agtatgagta ttcaacattt ccgtgtcgcc cttattccct 900
   tttttgcggc attttgcctt cctgtttttg ctcacccaga aacgctggtg aaagtaaaag 960
   atgctgaaga tcagttgggt gcacgagtgg gttacatcga actggatctc aacagcggta 1020
   agatccttga gagttttcgc cccgaagaac gttttccaat gatgagcact tttaaagttc 1080
   tgctatgtgg cgcggtatta tcccgtattg acgccgggca agagcaactc ggtcgccgca 1140
   tacactattc tcagaatgac ttggttgagt actcaccagt cacagaaaag catcttacgg 1200
   atggcatgac agtaagagaa ttatgcagtg ctgccataac catgagtgat aacactgcgg 1260
   ccaacttact tctgacaacg atcggaggac cgaaggagct aaccgctttt ttgcacaaca 1320
   tgggggatca tgtaactcgc cttgatcgtt gggaaccgga gctgaatgaa gccataccaa 1380
   acgacgagcg tgacaccacg atgcctgtag caatggcaac aacgttgcgc aaactattaa 1440
   ctggcgaact acttactcta gcttcccggc aacaattaat agactggatg gaggcggata 1500
   aagttgcagg accacttctg cgctcggccc ttccggctgg ctggtttatt gctgataaat 1560
   ctggagccgg tgagcgtggg tctcgcggta tcattgcagc actggggcca gatggtaagc 1620
   cctcccgtat cgtagttatc tacacgacgg ggagtcaggc aactatggat gaacgaaata 1680
   gacagatcgc tgagataggt gcctcactga ttaagcattg gtaactgtca gaccaagttt 1740
   actcatatat actttagatt gatttaaaac ttcattttta atttaaaagg atctaggtga 1800
   agatcctttt tgataatctc atgaccaaaa tcccttaacg tgagttttcg ttccactgag 1860
   cgtcagaccc cgtagaaaag atcaaaggat cttcttgaga tccttttttt ctgcgcgtaa 1920
   tctgctgctt gcaaacaaaa aaaccaccgc taccagcggt ggtttgtttg ccggatcaag 1980
   agctaccaac tctttttccg aaggtaactg gcttcagcag agcgcagata ccaaatactg 2040
   tccttctagt gtagccgtag ttaggccacc acttcaagaa ctctgtagca ccgcctacat 2100
   acctcgctct gctaatcctg ttaccagtgg ctgctgccag tggcgataag tcgtgtctta 2160
   ccgggttgga ctcaagacga tagttaccgg ataaggcgca gcggtcgggc tgaacggggg 2220
   gttcgtgcac acagcccagc ttggagcgaa cgacctacac cgaactgaga tacctacagc 2280
   gtgagctatg agaaagcgcc acgcttcccg aagggagaaa ggcggacagg tatccggtaa 2340
   gcggcagggt cggaacagga gagcgcacga gggagcttcc agggggaaac gcctggtatc 2400
   tttatagtcc tgtcgggttt cgccacctct gacttgagcg tcgatttttg tgatgctcgt 2460
   caggggggcg gagcctatgg aaaaacgcca gcaacgcggc ctttttacgg ttcctggcct 2520
   tttgctggcc ttttgctcac atgttctttc ctgcgttatc ccctgattct gtggataacc 2580
   gtattaccgc ctttgagtga gctgataccg ctcgccgcag ccgaacgacc gagcgcagcg 2640
   agtcagtgag cgaggaagcg gaaga 2665
<210> 2
<211> 5736
<212> DNA
<213> pRSVneo
<400> 2
   cttggaggtg cacaccaatg tggtgaatgg tcaaatggcg tttattgtat cgagctaggc 60
   acttaaatac aattatctct gcaatgcgga attcagtggt tcgtccaatc catgtcagac 120
   ctgtctgttg ccttcctaat aaggcacgat cgtaccacct tacttccacc aatcggcatg 180
   cacggtgctt tttctctcct tgtaaggcat gttgctaact catcgttacc atgttgcaag 240
   actacaagtg tattgcataa gactacattt ccccctccct atgcaaaagc gaaactacta 300
   tatcctgagg ggactcctaa ccgcgtacaa ccgaagcccc gcttttcgcc taaacacacc 360
   ctagtcccct cagatacgcg tatatctggc ccgtacatcg cgaagcagcg caaaacgcct 420
   aaccctaagc agattcttca tgcaattgtc ggtcaagcct tgccttgttg tagcttaaat 480
   tttgctcgcg cactactcag cgacctccaa cacacaagca gggagcagat actggcttaa 540
   ctatgcggca tcagagcaga ttgtactgag agtgcaccat atgcggtgtg aaataccgca 600
   cagatgcgta aggagaaaat accgcatcag gcgctcttcc gcttcctcgc tcactgactc 660
   gctgcgctcg gtcgttcggc tgcggcgagc ggtatcagct cactcaaagg cggtaatacg 720
   gttatccaca gaatcagggg ataacgcagg aaagaacatg tgagcaaaag gccagcaaaa 780
   ggccaggaac cgtaaaaagg ccgcgttgct ggcgtttttc cataggctcc gcccccctga 840
   cgagcatcac aaaaatcgac gctcaagtca gaggtggcga aacccgacag gactataaag 900
   ataccaggcg tttccccctg gaagctccct cgtgcgctct cctgttccga ccctgccgct 960
   taccggatac ctgtccgcct ttctcccttc gggaagcgtg gcgctttctc atagctcacg 1020
   ctgtaggtat ctcagttcgg tgtaggtcgt tcgctccaag ctgggctgtg tgcacgaacc 1080
   ccccgttcag cccgaccgct gcgccttatc cggtaactat cgtcttgagt ccaacccggt 1140
   aagacacgac ttatcgccac tggcagcagc cactggtaac aggattagca gagcgaggta 1200
   tgtaggcggt gctacagagt tcttgaagtg gtggcctaac tacggctaca ctagaaggac 1260
   agtatttggt atctgcgctc tgctgaagcc agttaccttc ggaaaaagag ttggtagctc 1320
   ttgatccggc aaacaaacca ccgctggtag cggtggtttt tttgtttgca agcagcagat 1380
   tacgcgcaga aaaaaaggat ctcaagaaga tcctttgatc ttttctacgg ggtctgacgc 1440
   tcagtggaac gaaaactcac gttaagggat tttggtcatg agattatcaa aaaggatctt 1500
   cacctagatc cttttaaatt aaaaatgaag ttttaaatca atctaaagta tatatgagta 1560
   aacttggtct gacagttacc aatgcttaat cagtgaggca cctatctcag cgatctgtct 1620
   atttcgttca tccatagttg cctgactccc cgtcgtgtag ataactacga tacgggaggg 1680
   cttaccatct ggccccagtg ctgcaatgat accgcgagac ccacgctcac cggctccaga 1740
   tttatcagca ataaaccagc cagccggaag ggccgagcgc agaagtggtc ctgcaacttt 1800
   atccgcctcc atccagtcta ttaattgttg ccgggaagct agagtaagta gttcgccagt 1860
   taatagtttg cgcaacgttg ttgccattgc tgcaggcatc gtggtgtcac gctcgtcgtt 1920
   tggtatggct tcattcagct ccggttccca acgatcaagg cgagttacat gatcccccat 1980
   gttgtgcaaa aaagcggtta gctccttcgg tcctccgatc gttgtcagaa gtaagttggc 2040
   cgcagtgtta tcactcatgg ttatggcagc actgcataat tctcttactg tcatgccatc 2100
   cgtaagatgc ttttctgtga ctggtgagta ctcaaccaag tcattctgag aatagtgtat 2160
   gcggcgaccg agttgctctt gcccggcgtc aacacgggat aataccgcgc cacatagcag 2220
   aactttaaaa gtgctcatca ttggaaaacg ttcttcgggg cgaaaactct caaggatctt 2280
   accgctgttg agatccagtt cgatgtaacc cactcgtgca cccaactgat cttcagcatc 2340
   ttttactttc accagcgttt ctgggtgagc aaaaacagga aggcaaaatg ccgcaaaaaa 2400
   gggaataagg gcgacacgga aatgttgaat actcatactc ttcctttttc aatattattg 2460
   aagcatttat cagggttatt gtctcatgag cggatacata tttgaatgta tttagaaaaa 2520
   taaacaaata ggggttccgc gcacatttcc ccgaaaagtg ccacctgacg tctaagaaac 2580
   cattattatc atgacattaa cctataaaaa taggcgtatc acgaggccct ttcgtcttca 2640
   agaattcctt tgcctaattt aaatgaggac ttaacctgtg gaaatatttt gatgtgggaa 2700
   gctgttactg ttaaaactga ggttattggg gtaactgcta tgttaaactt gcattcaggg 2760
   acacaaaaaa ctcatgaaaa tggtgctgga aaacccattc aagggtcaaa ttttcatttt 2820
   tttgctgttg gtggggaacc tttggagctg cagggtgtgt tagcaaacta caggaccaaa 2880
   tatcctgctc aaactgtaac cccaaaaaat gctacagttg acagtcagca gatgaacact 2940
   gaccacaagg ctgttttgga taaggataat gcttatccag tggagtgctg ggttcctgat 3000
   ccaagtaaaa atgaaaacac tagatatttt ggaacctaca caggtgggga aaatgtgcct 3060
   cctgttttgc acattactaa cacagcaacc acagtgcttc ttgatgagca gggtgttggg 3120
   cccttgtgca aagctgacag cttgtatgtt tctgctgttg acatttgtgg gctgtttacc 3180
   aacacttctg gaacacagca gtggaaggga cttcccagat attttaaaat tacccttaga 3240
   aagcggtctg tgaaaaaccc ctacccaatt tcctttttgt taagtgacct aattaacagg 3300
   aggacacaga gggtggatgg gcagcctatg attggaatgt cctctcaagt agaggaggtt 3360
   agggtttatg aggacacaga ggagcttcct ggggatccag acatgataag atacattgat 3420
   gagtttggac aaaccacaac tagaatgcag tgaaaaaaat gctttatttg tgaaatttgt 3480
   gatgctattg ctttatttgt aaccattata agctgcaata aacaagttaa caacaacaat 3540
   tgcattcatt ttatgtttca ggttcagggg gaggtgtggg aggtttttta aagcaagtaa 3600
   aacctctaca aatgtggtat ggctgattat gatctctagt caaggcacta tacatcaaat 3660
   attccttatt aaccccttta caaattaaaa agctaaaggt acacaatttt tgagcatagt 3720
   tattaatagc agacactcta tgcctgtgtg gagtaagaaa aaacagtatg ttatgattat 3780
   aactgttatg cctacttata aaggttacag aatatttttc cataattttc ttgtatagca 3840
   gtgcagcttt ttcctttgtg gtgtaaatag caaagcaagc aagagttcta ttactaaaca 3900
   cagcatgact caaaaaactt agcaattctg aaggaaagtc cttggggtct tctacctttc 3960
   tcttcttttt tggaggagta gaatgttgag agtcagcagt agcctcatca tcactagatg 4020
   gcatttcttc tgagcaaaac aggttttcct cattaaaggc attccaccac tgctcccatt 4080
   catcagttcc ataggttgga atctaaaata cacaaacaat tagaatcagt agtttaacac 4140
   attatacact taaaaatttt atatttacct tagagcttta aatctctgta ggtagtttgt 4200
   ccaattatgt cacaccacag aagtaaggtt ccttcacaaa gatccgggac caaagcggcc 4260
   atcgtgcctc cccactcctg cagttcgggg gcatggatgc gcggatagcc gctgctggtt 4320
   tcctggatgc cgacggattt gcactgccgg tagaactccg cgaggtcgtc cagcctcagg 4380
   cagcagctga accaactcgc gaggggatcg agcccggggt gggcgaagaa ctccagcatg 4440
   agatccccgc gctggaggat catccagccg gcgtcccgga aaacgattcc gaagcccaac 4500
   ctttcataga aggcggcggt ggaatcgaaa tctcgtgatg gcaggttggg cgtcgcttgg 4560
   tcggtcattt cgaaccccag agtcccgctc agaagaactc gtcaagaagg cgatagaagg 4620
   cgatgcgctg cgaatcggga gcggcgatac cgtaaagcac gaggaagcgg tcagcccatt 4680
   cgccgccaag ctcttcagca atatcacggg tagccaacgc tatgtcctga tagcggtccg 4740
   ccacacccag ccggccacag tcgatgaatc cagaaaagcg gccattttcc accatgatat 4800
   tcggcaagca ggcatcgcca tgggtcacga cgagatcctc gccgtcgggc atgcgcgcct 4860
   tgagcctggc gaacagttcg gctggcgcga gcccctgatg ctcttcgtcc agatcatcct 4920
   gatcgacaag accggcttcc atccgagtac gtgctcgctc gatgcgatgt ttcgcttggt 4980
   ggtcgaatgg gcaggtagcc ggatcaagcg tatgcagccg ccgcattgca tcagccatga 5040
   tggatacttt ctcggcagga gcaaggtgag atgacaggag atcctgcccc ggcacttcgc 5100
   ccaatagcag ccagtccctt cccgcttcag tgacaacgtc gagcacagct gcgcaaggaa 5160
   cgcccgtcgt ggccagccac gatagccgcg ctgcctcgtc ctgcagttca ttcagggcac 5220
   cggacaggtc ggtcttgaca aaaagaaccg ggcgcccctg cgctgacagc cggaacacgg 5280
   cggcatcaga gcagccgatt gtctgttgtg cccagtcata gccgaatagc ctctccaccc 5340
   aagcggccgg agaacctgcg tgcaatccat cttgttcaat catgcgaaac gatcctcatc 5400
   ctgtctcttg atcagatctt gatcccctgc gccatcagat ccttggcggc aagaaagcca 5460
   tccagtttac tttgcagggc ttcccaacct taccagaggg cgccccagct ggcaattccg 5520
   gttcgcttgc tgtccataaa accgcccagt ctagctatcg ccatgtaagc ccactgcaag 5580
   ctacctgctt tctctttgcg cttgcgtttt cccttgtcca gatagcccag tagctgacat 5640
   tcatccgggg tcagcaccgt ttctgcggac tggctttcta cgtgttccgc ttcctttagc 5700
   agcccttgcg ccctgagtgc ttgcggcagc gtgaag 5736
<210> 3
<211> 3584
<212> DNA
<213> pCRXA20
<400> 3
   gatatcatat tggctcatgt ccaacattac cgccatgttg acattgatta ttgactagtt 60
   attaatagta atcaattacg gggtcattag ttcatagccc atatatggag ttccgcgtta 120
   cataacttac ggtaaatggc ccgcctggct gaccgcccaa cgacccccgc ccattgacgt 180
   caataatgac gtatgttccc atagtagcgc caatagggac tttccattga cgtcaatggg 240
   tggagtattt acggtaaact gcccacttgg cagtacatca agtgtatcat atgccaagtc 300
   cgccccctat tgacgtcaat gacggtaaat ggcccgcctg gcattatgcc cagtacatga 360
   ccttacggga ctttcctact tggcagtaca tctacgtatt agtcatcgct attaccatgg 420
   tggatgcggt tttggcagta caccaatggg cgtggatagc ggtttgactc acggggattt 480
   ccaagtctcc accccattga cgtcaatggg agtttgtttg ggcaccaaaa tcaacgggac 540
   tttccaaaat gtcgtaataa ccccgccccg ttgacgcaaa tgggcggtag gcgtgtacgg 600
   tgggaggtct atataagcag agctcgttta gtgaaccgtc agatcgcctg gagacgccat 660
   ccacgctgtt ttgacctcca tagaagacac cgggaccgat ccagcctccg cggccgggaa 720
   cggtgcattg gaacgcggat tccccgtgcc aagagtgacg taagtaccgc ctatagactc 780
   tataggcaca cccctttggc tcttatgcat gctatactgt ttttggcttg gggcctatac 840
   acccccgctt ccttatgcta taggtgatgg tatagcttag cctataggtg tgggttattg 900
   accattattg accactcccc tattggtgac gatactttcc attactaatc cataacatgg 960
   ctctttgcca caactatctc tattggctat atgccaatac actgtccttt cgctcggcag 1020
   ctccttgctc ctaacagtgg aggccagact taggcacagc acaatgccca ccaccaccag 1080
   tgtgccacac aaggccgwgg cggtagggta tgtgtctgaa aatgagctcg gagattgggc 1140
   tcgcaccgct gacgcagatg gaagacttaa ggcagcggca gaagaagatg caggcagctg 1200
   agttgttgta ttctgataag agtcagaggt aactcccgtt gcggtgctgt taacggtgga 1260
   gggcagtgta gtctgagcag tactcgttgc tgccgcgcgc gccaccagac ataatagctg 1320
   acagactaac agactgttcc tttccatggg ttttttctgc agtcaccggt cgaccgaagc 1380
   ttcgcccggg cgggatcccg gcggccgccg gaattctgat cataatcagc cataccacat 1440
   ttgtagaggt tttacttgct ttaaaaaacc tcccacacct ccccctgaac ctgaaacata 1500
   aaatgaatgc aattgttgtt gttaacttgt ttattgcagc ttataatggt tacaaataaa 1560
   gcaatagcat cacaaatttc acaaataaag catttttttc actgcattct agttgtggtt 1620
   tgtccaaact catcaatgta tcttaggtac cacgtcaggt ggcacttttc ggggaaatgt 1680
   gcgcggaacc cctatttgtt tatttttcta aatacattca aatatgtatc cgctcatgag 1740
   acaataaccc tgataaatgc ttcaataata ttgaaaaagg aagagtatga ttgaacaaga 1800
   tggattgcac gcaggttctc cggccgcttg ggtggagagg ctattcggct atgactgggc 1860
   acaacagaca atcggctgct ctgatgccgc cgtgttccgg ctgtcagcgc aggggcgccc 1920
   ggttcttttt gtcaagaccg acctgtccgg tgccctgaat gaactgcagg acgaggcagc 1980
   gcggctatcg tggctggcca cgacgggcgt tccttgcgca gctgtgctcg acgttgtcac 2040
   tgaagcggga agggactggc tgctattggg cgaagtgccg gggcaggatc tcctgtcatc 2100
   tcaccttgct cctgccgaga aagtatccat catggctgat gcaatgcggc ggctgcatac 2160
   gcttgatccg gctacctgcc cattcgacca ccaagcgaaa catcgcatcg agcgagcacg 2220
   tactcggatg gaagccggtc ttgtcgatca ggatgatctg gacgaagagc atcaggggct 2280
   cgcgccagcc gaactgttcg ccaggctcaa ggcgcgcatg cccgacggcg aggatctcgt 2340
   cgtgacccat ggcgatgcct gcttgccgaa tatcatggtg gaaaatggcc gcttttctgg 2400
   attcatcgac tgtggccggc tgggtgtggc ggaccgctat caggacatag cgttggctac 2460
   ccgtgatatt gctgaagagc ttggcggcga atgggctgac cgcttcctcg tgctttacgg 2520
   tatcgccgct cccgattcgc agcgcatcgc cttctatcgc cttcttgacg agttcttctg 2580
   actcgaggcc agctgcatta atgaattggc ccacgcgcgg ggagaggcgg attgcgtatt 2640
   gggcgctctt ccgcttcctc gctcactgta ctcgctgcgc tcggtcgttc ggctgcggcg 2700
   agcggtatca gctcactcaa aggcggtaat acggttatcc acagaatcag gggataacgc 2760
   aggaaagaac atgtgagcaa aaggccagca aaaggccagg aaccgtaaaa aggccgcgtt 2820
   gctggcgttt ttccataggc tccgcccccc tgacgagcat cacaaaaatc gacgctcaag 2880
   tcagaggtgg cgaaacccga caggactata aagataccag gcgtttcccc ctggaagctc 2940
   cctcgtgcgc tctcctgttc cgaccctgcc gcttaccgga tacctgtccg cctttctccc 3000
   ttcgggaagc gtggcgcttt ctcatagctc acgctgtagg tatctcagtt cggtgtaggt 3060
   cgttcgctcc aagctgggct gtgtgcacga accccccgtt cagcccgacc gctgcgcctt 3120
   atccggtaac tatcgtcttg agtccaaccc ggtaagacac gacttatcgc cactggcagc 3180
   agccactggt aacaggatta gcagagcgag gtatgtaggc ggtgctacag agttcttgaa 3240
   gtggtggcct aactacggct acactagaag aacagtattt ggtatctgcg ctctgctgaa 3300
   gccagttacc ttcggaaaaa gagttggtag ctcttgatcc ggcaaacaaa ccaccgctgg 3360
   tagcggtggt ttttttgttt gcaagcagca gattacgcgc agaaaaaaag gatctcaaga 3420
   agatcctttg atcttttcta cggggtctga cgctcagtgg aacgaaaact cacgttaagg 3480
   gattttggtc atgagattat caaaaaggat cttcacctag atccttttaa attaaaaatg 3540
   aagttttaaa tcaatctaaa gtatatatga gtaaacttgg tctg 3584
<210> 4
<211> 2361
<212> DNA
<213> CMV_MIE_gene,_5'end-1
<400> 4
   ctgcagtgaa taataaaatg tgtgtttgtc cgaaatacgc gttttgagat ttctgtcgcc 60
   gactaaattc atgtcgcgcg atagtggtgt ttatcgccga tagagatggc gatattggaa 120
   aaatcgatat ttgaaaatat ggcatattga aaatgtcgcc gatgtgagtt tctgtgtaac 180
   tgatatcgcc atttttccaa aagtgatttt tgggcatacg cgatatctgg cgatacggct 240
   tatatcgttt acgggggatg gcgatagacg actttggcga cttgggcgat tctgtgtgtc 300
   gcaaatatcg cagtttcgat ataggtgaca gacgatatga ggctatatcg ccgatagagg 360
   cgacatcaag ctggcacatg gccaatgcat atcgatctat acattgaatc aatattggca 420
   attagccata ttagtcattg gttatatagc ataaatcaat attggctatt ggccattgca 480
   tacgttgtat ctatatcata atatgtacat ttatattggc tcatgtccaa tatgaccgcc 540
   atgttgacat tgattattga ctagttatta atagtaatca attacggggt cattagttca 600
   tagcccatat atggagttcc gcgttacata acttacggta aatggcccgc ctcgtgaccg 660
   cccaacgacc cccgcccatt gacgtcaata atgacgtatg ttcccatagt aacgccaata 720
   gggactttcc attgacgtca atgggtggag tatttacggt aaactgccca cttggcagta 780
   catcaagtgt atcatatgcc aagtccggcc ccctattgac gtcaatgacg gtaaatggcc 840
   cgcctggcat tatgcccagt acatgacctt acgggacttt cctacttggc agtacatcta 900
   cgtattagtc atcgctatta ccatggtgat gcggttttgg cagtacacca atgggcgtgg 960
   atagcggttt gactcacggg gatttccaag tctccacccc attgacgtca atgggagttt 1020
   gttttggcac caaaatcaac gggactttcc aaaatgtcgt aataaccccg ccccgttgac 1080
   gcaaatgggc ggtaggcgtg tacggtggga ggtctatata agcagagctc gtttagtgaa 1140
   ccgtcagatc gcctggagac gccatccacg ctgttttgac ctccatagaa gacaccggga 1200
   ccgatccagc ctccgcggcc gggaacggtg cattggaacg cggattcccc gtgccaagag 1260
   tgacgtaagt accgcctata gactctatag gcacacccct ttggctctta tgcatgctat 1320
   actgtttttg gcttggggcc tatacacccc cgctccttat gctataggtg atggtatagc 1380
   ttagcctata ggtgtgggtt attgaccatt attgaccact cccctattgg tgacgatact 1440
   ttccattact aatccataac atggctcttt gccacaacta tctctattgg ctatatgcca 1500
   atactctgtc cttcagagac tgacacggac tctgtatttt tacaggatgg ggtcccattt 1560
   attatttaca aattcacata tacaacaacg ccgtcccccg tgcccgcagt ttttattaaa 1620
   catagcgtgg gatctccacg cgaatctcgg gtacgtgttc cggacatggg ctcttctccg 1680
   gtagcggcgg agcttccaca tccgagccct ggtcccatgc ctccagcggc tcatggtcgc 1740
   tcggcagctc cttgctccta acagtggagg ccagacttag gcacagcaca atgcccacca 1800
   ccaccagtgt gccgcacaag gccgtggcgg tagggtatgt gtctgaaaat gagctcggag 1860
   attgggctcg caccgtgacg cagatggaag acttaaggca gcggcagaag aagatgcagg 1920
   cagctgagtt gttgtattct gataagagtc agaggtaact cccgttgcgg tgctgttaac 1980
   ggtggagggc agtgtagtct gagcagtact cgttgctgcc gcgcgcgcca ccagacataa 2040
   tagctgacag actaacagac tgttcctttc catgggtctt ttctgcagtc accgtccttg 2100
   acacgatgga gtcctctgcc aagagaaaga tggaccctga taatcctgac gagggccctt 2160
   cctccaaggt gccacggtac gtgtcggggt ttgtgccccc cctttttttt ataaaattgt 2220
   attaatgtta tatacatatc tcctgtatgt gacccatgtg cttatgactc tatttctcat 2280
   gtgtttaggc ccgagacacc cgtgaccaag gccacgacgt tcctgcagac tatgttgagg 2340
   aaggaggtta acagtcagct g                                           2361
<210> 5
   <211>
   <212> DNA
   <213> L523S-Adenovirus vector
<400> 5
<210> 6
   <211>
   <212> DNA
   <213> L523S
<400> 6
<210> 7
<211> 579
<212> prot
<213> L523S
<400> 7
   Met Asn Lys Leu Tyr Ile Gly Asn Leu Ser Glu Asn Ala Ala Pro Ser
   1               5                   10                  15
   Asp Leu Glu Ser Ile Phe Lys Asp Ala Lys Ile Pro Val Ser Gly Pro
               20                  25                  30
   Phe Leu Val Lys Thr Gly Tyr Ala Phe Val Asp Cys Pro Asp Glu Ser
           35                  40                  45
   Trp Ala Leu Lys Ala Ile Glu Ala Leu Ser Gly Lys Ile Glu Leu His
   50 55 60
   Gly Lys Pro Ile Glu Val Glu His Ser Val Pro Lys Arg Gln Arg Ile
   65 70 75 80
   Arg Lys Leu Gln Ile Arg Asn Ile Pro Pro His Leu Gln Trp Glu Val
   85 90 95
   Leu Asp Ser Leu Leu Val Gln Tyr Gly Val Val Glu Ser Cys Glu Gln
   100 105 110
   Val Asn Thr Asp Ser Glu Thr Ala Val Val Asn Val Thr Tyr Ser Ser
   115 120 125
   Lys Asp Gln Ala Arg Gln Ala Leu Asp Lys Leu Asn Gly Phe Gln Leu
   130 135 140
   Glu Asn Phe Thr Leu Lys Val Ala Tyr Ile Pro Asp Glu Thr Ala Ala
   145 150 155 160
   Gln Gln Asn Pro Leu Gln Gln Pro Arg Gly Arg Arg Gly Leu Gly Gln
   165 170 175
   Arg Gly Ser Ser Arg Gln Gly Ser Pro Gly Ser Val Ser Lys Gln Lys
   180 185 190
   Pro Cys Asp Leu Pro Leu Arg Leu Leu Val Pro Thr Gln Phe Val Gly
   195 200 205
   Ala Ile Ile Gly Lys Glu Gly Ala Thr Ile Arg Asn Ile Thr Lys Gln
   210 215 220
   Thr Gln Ser Lys Ile Asp Val His Arg Lys Glu Asn Ala Gly Ala Ala
   225 230 235 240
   Glu Lys Ser Ile Thr Ile Leu Ser Thr Pro Glu Gly Thr Ser Ala Ala
   245 250 255
   Cys Lys Ser Ile Leu Glu Ile Met His Lys Glu Ala Gln Asp Ile Lys
   260 265 270
   Phe Thr Glu Glu Ile Pro Leu Lys Ile Leu Ala His Asn Asn Phe Val
   275 280 285
   Gly Arg Leu Ile Gly Lys Glu Gly Arg Asn Leu Lys Lys Ile Glu Gln
   290 295 300
   Asp Thr Asp Thr Lys Ile Thr Ile Ser Pro Leu Gln Glu Leu Thr Leu
   305 310 315 320
   Tyr Asn Pro Glu Arg Thr Ile Thr Val Lys Gly Asn Val Glu Thr Cys
   325 330 335
   Ala Lys Ala Glu Glu Glu Ile Met Lys Lys Ile Arg Glu Ser Tyr Glu
   340 345 350
   Asn Asp Ile Ala Ser Met Asn Leu Gln Ala His Leu Ile Pro Gly Leu
   355 360 365
   Asn Leu Asn Ala Leu Gly Leu Phe Pro Pro Thr Ser Gly Met Pro Pro
   370 375 380
   Pro Thr Ser Gly Pro Pro Ser Ala Met Thr Pro Pro Tyr Pro Gln Phe
   385                 390                 395                 400
   Glu Gln Ser Glu Thr Glu Thr Val His Leu Phe Ile Pro Ala Leu Ser
                   405                 410                 415
   Val Gly Ala Ile Ile Gly Lys Gln Gly Gln His Ile Lys Gln Leu Ser
               420                 425                 430
   Arg Phe Ala Gly Ala Ser Ile Lys Ile Ala Pro Ala Glu Ala Pro Asp
           435                 440                 445
   Ala Lys Val Arg Met Val Ile Ile Thr Gly Pro Pro Glu Ala Gln Phe
       450                 455                 460
   Lys Ala Gln Gly Arg Ile Tyr Gly Lys Ile Lys Glu Glu Asn Phe Val
   465                 470                 475                 480
   Ser Pro Lys Glu Glu Val Lys Leu Glu Ala His Ile Arg Val Pro Ser
                   485                 490                 495
   Phe Ala Ala Gly Arg Val Ile Gly Lys Gly Gly Lys Thr Val Asn Glu
               500                 505                 510
   Leu Gln Asn Leu Ser Ser Ala Glu Val Val Val Pro Arg Asp Gln Thr
           515                 520                 525
   Pro Asp Glu Asn Asp Gln Val Val Val Lys Ile Thr Gly His Phe Tyr
       530                 535                 540
   Ala Cys Gln Val Ala Gln Arg Lys Ile Gln Glu Ile Leu Thr Gln Val
   545                 550                 555                 560
   Lys Gln His Gln Gln Gln Lys Ala Leu Gln Ser Gly Pro Pro Gln Ser
                   565                 570                 575
   Arg Arg Lys
<210> 8
   <211>
   <212> prot
   <213> L523S p13-21
<400> 8

## Description

### CROSS-REFERENCES TO RELATED APPLICATIONS

This application claims the benefit of U.S. Provisional Patent Application No. 60/477,232 filed June 9, 2003.

### BACKGROUND OF THE INVENTION

Despite enormous investments of financial and human resources, cancer remains one of the major causes of death. For example, cancers of the breast, lung, colon, prostate, bladder, and kidney as well as multiple hematological malignancies (*e.g*., adult and pediatric acute myeloid leukemia (AML), chronic myeloid leukemia (CML), acute lymphocytic leukemia (ALL), chronic lymphocytic leukemia (CLL) and secondary leukemia) lead to over six million deaths per year (*see, e.g.,* Wang et al., Oncogene 19:1519-1528 (2000)). Standard approaches to cure cancer have centered around a combination of surgery, radiation and chemotherapy. These approaches have resulted in some dramatic successes in certain malignancies. However, cancer is often incurable, when diagnosed beyond a certain stage. Alternative approaches to therapy are needed.

A common characteristic of malignancies is uncontrolled cell growth. Cancer cells appear to undergo a process of transformation from the normal phenotype to a malignant phenotype capable of autonomous growth. Amplification and overexpression of somatic cell genes (*i.e*., antigens) is considered to be a common primary event that results in the transformation of normal cells to malignant cells. The malignant phenotypic characteristics encoded by the oncogenic genes are passed on during cell division to the progeny of the transformed cells.

A number of antigens that are overexpressed in cancer have recently been identified (*see, e.g.,* Loeb et al., Cancer Res. 61:921-92 (2001); Xu et al., Cancer Res. 51:1563-1568 (2001); Gaiger et al., Proc. Amer. Assoc. Cancer Res. 946 (2002); Wang et al., Oncogene 19:1519-1528 (2000); Xu et al., Intl. J. Mol. Med. 8:S68 (2001); Wang et al., Oncogene 20:7699-709 (2001); Jager et al., Cancer Res. 61:2055-2061 (2001); Mhashilkar et al., Mol. Med. 7:271-282 (2001); Jiang et al., Amer. J. Surg. Path. 25:1397-1404 (2001); Fanger et al., Proc. Amer. Assoc. Cancer Res. 574 (2000); Calhoun et al., Proc. Amer. Assoc. Cancer Res. 437-438 (2000); Meagher et al., Proc. Amer. Assoc. Cancer Res. 173 (2000); Xu et al., Cancer Res. 60:1677-1682 (2000); Wang et al., Oncogene 19:1519-1528 (2000); Villaret et al., Laryngoscope 110:374-381 (2000); Bangur et al., Proc. Amer. Assoc. Cancer Res. 680 (2000); Henderson et al., Immunol. Invest. 29:87-91 (2000); Pyle et al., Proc. Amer. Assoc. Cancer Res. 679 (2000); Mayo et al., EMBO 18(14):3990-4003 (1999)). Some of these antigens have been identified as oncogenes, i.e., genes operative in malignant cells and responsible for, or associated with, transformation and include, *e.g*., Wilms' tumor suppressor gene (WT1) and B726P, both which are overexpressed in breast tumors; L523S which is overexpressed in lung squamous cell carcinoma, and P501 S which is overexpressed in prostate tumors (*see, e.g.,* Xu et al., Cancer Res. 61:1563-68, 2001)). These antigens can conveniently be used for diagnosis and therapy of cancers. For example, these antigens can conveniently be used to generate cancer specific immune responses. In particular, WT1 polypeptides have been used in protein based vaccines (*see, e.g.,* Gaiger et al., Proc. Amer. Assoc. Cancer Res. 42:698 (2001)).

Immunization with nucleic acid (*i.e*., genetic immunization) is a promising technology that may advance vaccine efficacy and safety (*see, e.g.,* Cui et al., J. Biotechnol. 102(2):105-115 (2003); Robinson and Torres, Semin. Immunol. 9:271 (1997); Robinson et al., Int. J. Mol. Med. 4:549 (1999); and Hartikka et al., Hum. Gene Ther. 7:1205-1217 (1996)). There are several well-characterized advantages of nucleic acid vaccines compared to protein-based or live-attenuated viral vaccines. A hallmark of DNA vaccination is induction of cytotoxic T lymphocyte (CTL) activity that is superior to protein vaccination. Cellular immunity can be enhanced because the protein being encoded by the nucleic acid is processed and presented in a way that is analogous to the processing of viral antigens *(see, e.g.,* Corr et al., J. Exp. Med. 184:1555 (1996)). In addition, plasmid-based vectors are easier to manufacture than protein-based or whole, live organism vaccines, and are considered safer to use. Finally, it is expected that it will be easier to incorporate new or altered antigens in DNA vaccines.

Protective immunity following DNA vaccination has been demonstrated in a variety of mouse models (*see, e.g.,* Wang et al., Vaccine; 21(15):1672-80 (2003);

Manickan et al., J. Immunol. 155:259 (1995); Zinckgraf et al., Vaccine 21(15):1640-9 (2003); Ott et al., J Control Release 79(1-3):1-5 (2002); Fynan et al., Proc. Natl. Acad. Sci. USA 90:11478 (1993)). However, reports from human studies have been less encouraging (*see,* e.g., Denis-Mize et al., Gene Ther. 7:2105 (2000)).

Thus, there is a need for expression vectors to express heterologous nucleic acid sequences that may be used as components of compositions and methods for stimulating immunogenic responses against antigens overexpressed in cancer cells. In particular there is a need for compositions that can target a malignancy with which a particular cancer antigen is associated, and for compositions and methods that can elicit and enhance an immune response to the particular cancer antigen. The present invention fulfills this and other needs.

### BRIEF SUMMARY OF THE INVENTION

The present invention provides expression vectors and methods for expression heterologous nucleic acid sequences using such expression vectors. Within one embodiment the expression vector comprises an expression cassette comprising from 5' to 3' the following elements: a CMV promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence. In some embodiments, the CMV intron A sequence has a deletion from about base 1513 to about base 1736. In other embodiments, the heterologous nucleic acid encodes a cancer antigen, such as, for example,L523S (SEQ ID NO:6). In some embodiments the expression cassette comprises nucleotides 54-3675 of the sequence set forth in SEQ ID NO:3, nucleotides 1-1653 of the sequence set forth in SEQ ID NO:3, or the nucleotide sequence set forth in SEQ ID NO:3. The invention also provides host cells comprising the expression vectors described above. In some embodiments, the host cell is *E*. *coli* or mammalian cells. The invention further provides immunogenic compositions comprising the expression vector described above.

Another embodiment of the invention provides a methods for expressing a heterologous nucleic acid sequence. A host cell comprising an expression vector comprising an expression cassette comprising from 5' to 3' the following elements: a CMV promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence is cultured. In some embodiments, the CMV intron A sequence has a deletion from about base 1513 to about base 1736. In some embodiments, the heterologous nucleic acid encodes a cancer antigen, such as, for example,L523S (SEQ ID NO:6). In some embodiments, the expression cassette comprises nucleotides 54-3675 of the sequence set forth in SEQ ID NO:3, nucleotides 1-1653 of the sequence set forth in SEQ ID NO:3, or the nucleotide sequence set forth in SEQ ID NO: 3. In some embodiments, the expression cassette comprises the nucleotides set forth in SEQ ID NO:4. In some embodiments, the host cell is *E*. *coli* or mammalian cells.

A further embodiment of the invention provides a method for eliciting an immune response, the method comprising the steps of administering an immunogenically effective amount of the immunogenic composition comprising a vector comprising an expression cassette comprising from 5' to 3' the following elements: a CMV promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence and wherein the immune response is directed against a peptide encoded by the heterologous nucleic acid sequence. In some embodiments, the immunogenic composition is administered multiple times.

### BRIEF DESCRIPTION OF THE SEQUENCES

SEQ ID NO: 1 is a DNA sequence for pUC9.

SEQ ID NO:2 is a DNA sequence for pRSVneo.

SEQ ID NO:3 is a DNA sequence for pCRXA20.

SEQ ID NO:4 is a DNA sequence for a CMV_MIE_gene,_5'end.

SEQ ID NO:5 is a DNA sequence for a adenoviral vector comprising a L523S sequence.

SEQ ID NO:6 is a DNA sequence for L523S.

SEQ ID NO:7 is a polypeptide sequence for L523S.

SEQ ID NO:8 is a polypeptide sequence for L523S p13-21.

### DETAILED DESCRIPTION OF THE INVENTION

### I. Introduction

The present invention provides compositions that can target a malignancy with which a particular cancer antigen is associated, and compositions and methods that can elicit and enhance an immune response to the particular cancer antigen. In particular, the invention provides an expression vector DNA plasmid that can express genes when transfected into eukaryotic cells. The vector comprises an expression cassette comprising from 5' to 3' the following elements: a CMV promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence. In an exemplary embodiment, the heterologous nucleic acid sequence is a cancer antigen. In some embodiments, the heterologous nucleic acid sequence encodes a cancer antigen. The invention also provides compositions comprising the expression vector and pharmaceutically acceptable carrier. The invention further provides compositions for expressing the heterologous nucleic acid sequence and methods for eliciting an immune response against the polypeptide encoded by the heterologous nucleic acid sequence by administering such compositions to a subject.

### II. Definitions

An "immunogenic composition" is one that elicits or modulates an immune response, preferably the composition induces or enhances an immune response in response to a particular antigen. Immune responses include humoral immune responses and cell-mediated immune responses. An immunogenic composition can be used therapeutically or prophylactically to treat or prevent disease at any stage.

As used herein, "heterologous" is defined in relation to a predetermined referenced nucleic acid or amino acid sequence. For example, with respect to a structural gene sequence, a heterologous promoter is defined as a promoter which does not naturally occur adjacent to the referenced structural gene, but which is positioned by laboratory manipulation. Likewise, a heterologous gene or nucleic acid segment is defined as a gene or segment that does not naturally occur adjacent to the referenced promoter and/or enhancer elements. With respect to polypeptide sequences, *i.e*., polypeptide sequences encoding proteins, a heterologous peptide is one which does not naturally occur adjacent to the referenced protein or portion thereof. In some embodiments, the heterologous polypeptide is a proteins, polypeptides, or fusion proteins.

A "proteins, polypeptides, or fusion proteins" refers to a protein having at least two polypeptides covalently linked, in which one polypeptide comes from one protein sequence or domain and the other polypeptide comes from another protein sequence or domain. The polypeptides can be linked either directly or via a covalent linker, *e.g*., an amino acid linker, such as a polyglycine linker, or another type of chemical linker, *e.g.*, a carbohydrate linker, a lipid linker, a fatty acid linker, a polyether linker, *e.g*., PEG, etc. (see, *e.g*., Hermanson (1996) Bioconjugate techniques). The polypeptides forming the proteins, polypeptides, or fusion proteins are typically linked C-terminus to N-terminus, although they can also be linked C-terminus to C-terminus, N-terminus to N-terminus, or N-terminus to C-terminus. The polypeptides of the proteins, polypeptides, or fusion proteins can be in any order. The term "proteins, polypeptides, or fusion proteins" also refers to conservatively modified variants, polymorphic variants, alleles, mutant, subsequences and interspecies homologues of the polypeptides that make up the proteins, polypeptides, or fusion proteins proteins, polypeptides, or fusion proteins may be produced by covalently linking a chain of amino acids from one protein sequence to a chain of amino acids from another protein sequence, *e.g*., by preparing a recombinant polynucleotide contiguously encoding the proteins, polypeptides, or fusion proteins proteins, polypeptides, or fusion proteins can comprise 2, 3, 4 or more different chains of amino acids from the same or different species. The different chains of amino acids in a proteins, polypeptides, or fusion proteins may be directly spliced together or may be indirectly spliced together via a chemical linking group or an amino acid linking group. The proteins, polypeptides, or fusion proteins may optionally comprise other components, as described in more detail herein.

An "adjuvant" is a non-specific immune response enhancer.

"Humoral immune responses" are mediated by cell free components of the blood, *i.e.,* plasma or serum; transfer of the serum or plasma from one individual to another transfers immunity.

"Cell mediated immune responses" are mediated by antigen specific lymphocytes; transfer of the antigen specific lymphocytes from one individual to another transfers immunity.

The immunogenic compositions of the present invention are administered to a subject in an amount sufficient to elicit a therapeutic or prophylactic response in the subj ect. An amount adequate to accomplish this is defined as "therapeutically effective dose or amount."

The immunogenic compositions of the present invention are administered to a subject in an amount sufficient to elicit an immune response in the subject. An amount adequate to accomplish this is defined as "immunogenically effective dose or amount."

The term "protein" is used herein interchangeably with "polypeptide" and "peptide."

"Nucleic acid" refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (*e.g*., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al. (1991) Nucleic Acid Res. 19:5081; Ohtsuka et al. (1985) J. Biol. Chem. 260:2605-2608; Rossolini et al. (1994) Mol. Cell. Probes 8:91-98). The term nucleic acid is used interchangeably with gene, cDNA, mRNA, oligonucleotide, and polynucleotide.

A polynucleotide sequence comprising a proteins, polypeptides, or fusion proteins of the invention hybridizes under stringent conditions to each of the nucleotide sequences encoding each individual polypeptide of the proteins, polypeptides, or fusion proteins. The polynucleotide sequences encoding the individual polypeptides of the fusion polypeptide therefore include conservatively modified variants, polymorphic variants, alleles, mutants, subsequences, and interspecies homologs.

"Percentage of sequence identity" is determined by comparing two optimally aligned sequences over a comparison window, wherein the portion of the polynucleotide sequence in the comparison window may comprise additions or deletions (*i.e*., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The term "substantial identity" of polynucleotide sequences means that a polynucleotide comprises a sequence that has at least 25% sequence identity. Alternatively, percent identity can be any integer from 25% to 100%. More preferred embodiments include at least: 25%, 30%, 35%, 40%, 45%, 50%, 55%, 60%, 65%, 70%, 75%, 80%, 85%, 90%, 95%, or 99% or higher, compared to a reference sequence using the programs described herein, preferably BLAST using standard parameters, as described below. One of skill will recognize that these values can be appropriately adjusted to determine corresponding identity of proteins encoded by two nucleotide sequences by taking into account codon degeneracy, amino acid similarity, reading frame positioning and the like. "Substantial identity" of amino acid sequences for these purposes normally means that a polypeptide comprises a sequence that has at least 40% sequence identity to, e.g., SEQ ID NO:3. Preferred percent identity of polypeptides can be any integer from 40% to 100%. More preferred embodiments include at least 60%, 65%, 70%, 75%, 80%, 85%, 90%, 95%, or 99%. Polypeptides which are "substantially similar" share sequences as noted above except that residue positions which are not identical may differ by conservative amino acid changes. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, aspartic acid-glutamic acid, and asparagine-glutamine.

Optimal alignment of sequences for comparison may be conducted by the local identity algorithm of Smith and Waterman (1981) Add. APL. Math. 2:482, by the identity alignment algorithm of Needleman and Wunsch (1970) J. Mol. Biol. 48:443, by the search for similarity method of Pearson and Lipman (1988) Proc. Natl. Acad. Sci. U.S.A. 85:2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

A preferred example of algorithms that are suitable for determining percent sequence identity and sequence similarity are the BLAST and BLAST 2.0 algorithms, which are described in Altschul et al. (1977) Nuc. Acids Res. 25:3389-3402 and Altschul et al. (1990) J. Mol. Biol. 215:403-410, respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff (1989) Proc. Natl. Acad. Sci. USA 89:10915) alignments (B) of 50, expectation (E) of 10, M=5, N=-4, and a comparison of both strands.

Another indication that nucleotide sequences are substantially identical is if two molecules hybridize to each other, or to a third nucleic acid, under moderately, and preferably highly, stringent conditions. Stringent conditions are sequence dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (*e.g*., 10 to 50 nucleotides) and at least about 60°C for long probes (*e.g.*, greater than 50 nucleotides). Stringent conditions may also be achieved with the addition of destabilizing agents such as formamide. For selective or specific hybridization, a positive signal is at least two times background, preferably 10 times background hybridization.

Exemplary stringent hybridization conditions can be as following: 50% formamide, 5X SSC, and 1% SDS, incubating at 42°C, or, 5X SSC, 1% SDS, incubating at 65°C, with wash in 0.2X SSC, and 0.1% SDS at 65°C.

For the purpose of the invention, suitable "moderately stringent conditions" include, for example, prewashing in a solution of 5X SSC, 0.5% SDS, 1.0 mM EDTA (pH 8.0), hybridizing at 50°C-65°C, 5X SSC overnight, followed by washing twice at 65°C for 20 minutes with each of 2X, 0.5X and 0.2X SSC (containing 0.1% SDS). Such hybridizing DNA sequences are also within the scope of this invention.

"Proliferation of T cells," as described herein, includes the multiplication of T cells as well as the stimulation of T cells leading to multiplication, i.e., the initiation of events leading to mitosis and mitosis itself. Methods for detecting proliferation of T cells are discussed below.

### III. Vectors of the Invention

The present invention provides compositions that can target a malignancy with which a particular cancer antigen is associated, and compositions and methods that can elicit and enhance an immune response to the particular cancer antigen. In particular, the invention provides an expression cassette comprising from 5' to 3' the following elements: a CMV promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence.

As disclosed within the present invention, the protein expression product of nucleic acid encoding the heterologous polypeptide is recognized by T cells.

### A. Expression Vectors

In one embodiment, the compounds of the present invention comprise expression vectors comprising nucleic acids encoding proteins, polypeptides, or fusion proteins comprising the heterologous polypeptides or variants of the heterologous polypeptides. In the proteins, polypeptides, or fusion proteins of the invention, the heterologous polypeptide component can be directly fused or fused via a linker, *e.g*., an amino acid linker or another type of chemical linker to another polypeptide. Other variants within the scope of the invention include proteins, polypeptides, or fusion proteins in which the primary amino acid structure of native heterologous polypeptide is modified by forming covalent or aggregative conjugates with other peptides or polypeptides, or chemical moieties such as glycosyl groups, lipids, phosphate, acetyl groups and the like. Covalent derivatives may be prepared, for example, by linking particular functional groups to amino acid side chains or at the N- or C-terminus.

The present invention also includes expression cassettes encoding fusion proteins and fusion proteins with or without glycosylation. Fusion proteins expressed in yeast or mammalian expression systems may be similar to, or slightly different in molecular weight and glycosylation pattern from, the native molecules, depending upon the expression system. Expression of DNA encoding polypeptides in bacteria such as *E*. *coli* typically provides non-glycosylated molecules. N-glycosylation sites of eukaryotic proteins are characterized by the amino acid triplet Asn-A₁-Z, wherein A₁ is any amino acid except Pro, and Z is Ser or Thr. Variants of proteins, polypeptides, or fusion proteins having inactivated N-glycosylation sites can be produced by techniques known to one skilled in the art, such as oligonucleotide synthesis and ligation or site-specific mutagenesis techniques, and are within the scope of the invention. Alternatively, N-linked glycosylation sites can be added to a protein, polypeptide, or fusion protein.

The proteins, polypeptides, or fusion proteins of the present invention, which will be understood to include variants, include any possible combination between human and non-human polypeptides. Non-human polypeptides comprise polypeptides from any mammal, such as, *e.g.,* rat, mouse, guinea pig, horse, cow, pig, sheep, dog, *etc.*

Any variants of the proteins, polypeptides, or fusion proteins of the present invention are included as embodiments of the present invention. In one embodiment, such variants are substantially identical or substantially similar to the native protein and retain the ability to stimulate an immune response. The effect of any sequence modification on the ability of a protein to produce an immune response may be readily determined, for example, by analyzing the ability of the mutated protein to induce a T cell response using, for example, the methods described herein, or by analyzing the ability of the mutated protein to produce antibodies.

Within certain specific embodiments, the proteins, polypeptides, or fusion proteins of the invention may comprise a fusion partner, such as, *e.g*., an immunological fusion partner or an expression enhancer. A fusion partner may, for example, assist in providing T helper epitopes (an immunological fusion partner), preferably T helper epitopes recognized by humans, or may assist in expressing the proteins, polypeptides, or fusion proteins (an expression enhancer) at higher yields than the recombinant proteins, polypeptides, or fusion proteins. Certain preferred fusion partners are both immunological and expression enhancing fusion partners. Other fusion partners may be selected so as to increase the solubility of the proteins, polypeptides, or fusion proteins or to enable the proteins, polypeptides, or fusion proteins to be targeted to desired intracellular compartments. Still further fusion partners include affinity tags, which facilitate the purification of the proteins, polypeptides, or fusion proteins of interest.

Also provided are proteins, polypeptides, or fusion proteins that comprise a fusion polypeptide as described herein together with an unrelated immunogenic protein. Preferably, the immunogenic protein is capable of eliciting a recall response. Examples of such proteins include tetanus, tuberculosis and hepatitis proteins (*see, e.g*., Stoute et al. (1997) New Engl. J. Med. 336:86-91).

In other embodiments, an immunological fusion partner is derived from protein D, a surface protein of the gram-negative bacterium *Haemophilus influenza* B (WO 91/18926). Preferably, a protein D derivative comprises approximately the first third of the protein (*e.g*., the first N-terminal 100-110 amino acids), and the protein D derivative may be lipidated. Within certain preferred embodiments, the first 109 residues of a Lipoprotein D fusion partner are included on the N-terminus to provide the proteins, polypeptides, or fusion proteins with additional exogenous T-cell epitopes and to increase the expression level in *E*. *coli* (thus functioning as an expression enhancer). The lipid tail ensures optimal presentation of the proteins, polypeptides, or fusion proteins to antigen presenting cells. Other fusion partners include the non-structural protein from influenzae virus, NS1 (hemaglutinin), or an immunogenic portion thereof *(see, e.g.,* WO 99/40188 and WO 93/04175). Typically, the N-terminal 81 amino acids are used, although different fragments that include T-helper epitopes may be used. In one embodiment, the proteins, polypeptides, or fusion proteins of the present invention further comprise the NS1 fusion partner or an immunogenic fragment thereof, preferably attached to the N-terminal region of the proteins, polypeptides, or fusion proteins. The proteins, polypeptides, or fusion proteins comprising the NS1 partner are preferably recombinantly expressed in BHK cells, as described *infra.*

In another embodiment, the immunological fusion partner is the protein known as LYTA, or a portion thereof (preferably a C-terminal portion). LYTA is derived from *Streptococcus pneumonia,* which synthesizes an N-acetyl-L-alanine amidase known as amidase LYTA (encoded by the LytA gene; Gene 43:265-292 (1986)). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E*. *coli* C-LYTA expressing plasmids useful for expression of proteins, polypeptides, or fusion proteins. Purification of hybrid proteins containing the C-LYTA fragment at the amino terminus has been described (*see,* Biotechnology 10:795-798 (1992)). Within a preferred embodiment, a repeat portion of LYTA may be incorporated into a proteins, polypeptides, or fusion proteins. A repeat portion is found in the C-terminal region starting at residue 178. A particularly preferred repeat portion incorporates residues 188-305.

Nucleic acids encoding the fusion partner polypeptides of this invention can be prepared by any suitable method known in the art. Exemplary methods include cloning and restriction of appropriate sequences or direct chemical synthesis by methods such as the phosphotriester method of Narang et al. (1979) Meth. Enzymol. 68:90-99; the phosphodiester method of Brown et al. (1979) Meth. Enzymol. 68:109-151; the diethylphosphoramidite method of Beaucage et al. (1981) Tetra. Lett. 22:1859-1862; and the solid support method of U.S. Patent No. 4,458,066.

Recombinant nucleic acids that encode a fusion polypeptide comprising a fusion partner polypeptide and a selected proteins, polypeptides, or fusion proteins can be prepared using any methods known in the art. As described above, recombinant nucleic acids are constructed so that, preferably, the fusion partner polynucleotide sequence is located 5' to the polynucleotide sequence encoding the proteins, polypeptides, or fusion proteins of interest. The fusion partner and proteins, polypeptides, or fusion proteins polynucleotide sequences can also be modified to facilitate their fusion and subsequent expression.

The recombinant nucleic acids can further comprise other nucleotide sequences such as sequences that encode affinity tags to facilitate protein purification protocol.

### B. Variants of the Proteins Encoded by the Heterologous Nucleic Acids

In general, CD4⁺ T cell populations are considered to function as helpers or inducers through the release of lymphokines when stimulated by a specific antigen; however, a subset of CD4⁺ cells can act as cytotoxic T lymphocytes (CTL). Similarly, CD8⁺ T cells are considered to function by directly lysing antigenic targets; however, under a variety of circumstances they can secrete lymphokines to provide helper or DTH function. Despite the potential of overlapping function, the phenotypic CD4 and CD8 markers are linked to the recognition of peptides bound to class I or class II MHC antigens. The recognition of an antigen in the context of class I or class II MHC mandates that CD4⁺ and CD8⁺ T cells respond to different antigens or to the same antigen presented under different circumstances. The binding of immunogenic peptides to class II MHC antigens most commonly occurs for antigens ingested by antigen presenting cells.

CD4⁺ T cells generally recognize antigens that have been external to the cancer cells. By contrast, under normal circumstances, binding of peptides to class I MHC occurs only for proteins present in the cytosol and synthesized by the target itself, proteins in the external environment are excluded. An exception to this is the binding of exogenous peptides with a precise class I binding motif which are present outside the cell in high concentration. Thus, CD4⁺ and CD8⁺ T cells have broadly different functions and tend to recognize different antigens as a reflection of where the antigens normally reside.

Another way to make amino acid substitutions to produce variants of the present invention is to identify and replace amino acids in T cell motifs with potential to bind to class II MHC molecules (for CD4⁺ T cell response) or class I MHC molecules (for CD8⁺ T cell response). Peptide segments with a motif with theoretical potential to bind to class II MHC molecules may be identified by computer analysis. For example, a protein sequence analysis package, *T Sites,* that incorporates several computer algorithms designed to distinguish potential sites for T cell recognition can be used (Feller et al. (1991) Nature 349:720-721). Two searching algorithms are used: (1) the AMPHI algorithm described by Margalit (Feller et al. (1991) Nature 349:720-721; Margalit et al. (1987) J. Immunol. 138:2213-2229) identifies epitope motifs according to alpha-helical periodicity and amphipathicity; (2) the Rothbard and Taylor algorithm identifies epitope motifs according to charge and polarity pattern (Rothbard et al. (1988) EMBO J. 7:93-100). Segments with both motifs are most appropriate for binding to class II MHC molecules. CD8⁺ T cells recognize peptides bound to class I MHC molecules. Parker et al. (1994) J. Immunol. 152:163 have determined that peptides binding to particular MHC molecules share discernible sequence motifs. A peptide motif for binding in the groove of HLA-A2.1 has been defined by Edman degradation of peptides stripped from HLA-A2.1 molecules of a cultured cell line (Table 1, from Falk et al. (1991) Nature 351:290-296). The method identified the typical or average HLA-A2.1 binding peptide as being 9 amino acids in length with dominant anchor residues occurring at positions 2 (L) and 9 (V). Commonly occurring strong binding residues have been identified at positions 2 (M), 4 (E,K), 6 (V), and 8 (K). The identified motif represents the average of many binding peptides.

**Table 1: The HLA-A2.1 Restricted Motif**

| | Amino Acid Position | | | | | | | | | Point |
|---|---|---|---|---|---|---|---|---|---|---|
| | | | | | | | | | | Assignment |
| | 1 | 2 | 3 | 4 | 5 | 6 | 7 | 8 | 9 | |
| Dominant Binding | | L | | | | | | | V | +3 |
| Anchor Residue | | | | | | | | | | |
| Strong Binding | | M | | E | | V | | K | | +2 |
| Residue | | | | K | | | | | | |
| Weak Binding | I | | A | G | I | I | A | E | L | +1 |
| Residue | L | | Y | P | K | L | Y | S | | |
| | F | | F | D | Y | T | H | | | |
| | K | | P | T | N | | | | | |
| | M | | M | | G | | | | | |
| | Y | | | | | | | | | |
| | | | S | | V | | | | | |
| | | | | | H | | | | | |

The derived peptide motif as currently defined is not particularly stringent. Some HLA-A2.1 binding peptides do not contain both dominant anchor residues and the amino acids flanking the dominant anchor residues play major roles in allowing or disallowing binding. Not every peptide with the current described binding motif will bind, and some peptides without the motif will bind. However, the current motif is valid enough to allow identification of some peptides capable of binding. Of note, all MHC molecules and respective motifs place 6 amino acids between the dominant anchor amino acids at residues 2 and 9.

Following identification of peptide motifs within proteins, amino acid substitutions can be made conservatively or non-conservatively. The latter type of substitutions are intended to produce an improved protein that is more potent and/or more broadly cross-reactive. An example of a more potent protein or peptide is one that binds with higher affinity to the same MHC molecule as the natural protein or polypeptide, without affecting recognition by T cells specific for natural protein or polypeptide. An example of a polypeptide with broader cross-reactivity is one that induces more broadly cross-reactive immune responses (*i.e*., binds to a greater range of MHC molecules) than natural polypeptide. Similarly, one or more amino acids residing between peptide motifs and having a spacer function (*e.g*., do not interact with a MHC molecule or T cell receptor) can be substituted conservatively or non-conservatively. It will be evident to one of ordinary skill in the art that polypeptides containing one or more amino acid substitutions can be tested for beneficial or adverse immunological interactions by a variety of assays, including those described herein for the ability to stimulate T cell recognition.

Variants within the scope of this invention can also, or alternatively, contain other modifications, including the deletion or addition of amino acids, that have minimal influence on the desired immunological properties of the polypeptide, as described *supra.* It will be appreciated by one of ordinary skill in the art that truncated forms or non-native extended forms of a protein can be used, provided the desired immunological properties are at least roughly equivalent to that of full length, native protein. Cysteine residues may be deleted or replaced with other amino acids to prevent formation of incorrect intramolecular disulfide bridges upon renaturation. Other approaches to mutagenesis involve modification of adjacent dibasic amino acid residues to enhance expression in yeast systems in which KEX2 protease activity is present.

### IV. Preparing Polynucleotides of The Invention

### A. Polynucleotides Encoding Proteins, polypeptides, or fusion proteins

In accordance with the invention, any nucleotide sequence which encodes the amino acid sequence of the proteins, polypeptides, or fusion proteins of interest can be used to generate recombinant molecules which direct the expression of the proteins, polypeptides, or fusion proteins.

In order to clone full-length coding sequences or homologous variants to generate the fusion polynucleotides, labeled DNA probes designed from any portion of a nucleotide sequences or its complements may be used to screen a genomic or cDNA library, to identify the coding sequence of each individual component of the proteins, polypeptides, or fusion proteins. The nucleotide sequences can be from any suitable mammal, *e.g*., human, rat, mouse, horse, cow, pig, sheep, dog, *etc.*

Such clones may be isolated by screening an appropriate expression library for clones that express a full length protein of interest, such as a cancer antigen. The library preparation and screen may generally be performed using methods known to one of ordinary skill in the art, such as methods described in Sambrook et al., Molecular Cloning: A Laboratory Manual, Cold Spring Harbor Laboratories, Cold Spring Harbor, NY (1989). Briefly, a bacteriophage expression library may be plated and transferred to filters. The filters may then be incubated with a detection reagent. In the context of this invention, a "detection reagent" is any compound capable of binding to the protein, which may then be detected by any of a variety of means known to one of ordinary skill in the art. Typical detection reagents contain a "binding agent," such as Protein A, Protein G, IgG or a lectin, coupled to a reporter group. Preferred reporter groups include enzymes, substrates, cofactors, inhibitors, dyes, radionuclides, luminescent groups, fluorescent groups and biotin. More preferably, the reporter group is horseradish peroxidase, which may be detected by incubation with a substrate such as tetramethylbenzidine or 2,2'-azino-di-3-ethylbenz-thiazoline sulfonic acid. For example, plaques containing genomic or cDNA sequences that express a cancer antigen are isolated and purified by techniques known to one of ordinary skill in the art. Appropriate methods may be found, for example, in Sambrook *et al., supra.*

Isolation of coding sequences may also be carried out by the polymerase chain reaction (PCR) using two degenerate oligonucleotide primer pools designed on the basis of the coding sequences disclosed herein. The desired nucleic acids can also be cloned using other well known amplification techniques. Examples of protocols sufficient to direct persons of skill through *in vitro* amplification methods, including PCR, ligase chain reaction (LCR), Qβ-replicase amplification and other RNA polymerase mediated techniques are found in Sambrook *et al., supra,* and Ausubel et al. Current Protocols in Molecular Biology (1994), as well as in U.S. Patent No. 4,683,202; PCR Protocols A Guide to Methods and Applications (Innis et al. eds. 1990); Arnheim & Levinson C&ENpp. 36-47 (October 1, 1990); The Journal of NIH Research 3:81-94 (1991); Kwoh et al. (1989) Proc. Natl. Acad. Sci. USA 86:1173; Guatelli et al. (1990) Proc. Natl. Acad. Sci. USA 87:1874; Lomell et al. (1989) J. Clin. Chem. 35:1826; Landegren et al. (1988) Science 241:1077-1080; Van Brunt (1990) Biotechnology 8:291-294; Wu et al. (1989) Gene 4:560; and Barringer et al. (1990) Gene 89:117. Improved methods of cloning *in vitro* amplified nucleic acids are described in U.S. Patent No. 5,426,039. Suitable primers for use in the amplification of the nucleic acids of the invention can be designed based on the sequences provided herein.

In accordance with the invention, a polynucleotide of the invention which encodes a proteins, polypeptides, or fusion proteins, fragment thereof, or functional equivalent thereof may be used to generate recombinant nucleic acid molecules that direct the expression of the proteins, polypeptides, or fusion proteins, fragment thereof, or functional equivalent thereof, in appropriate host cells. The fusion polypeptide products encoded by such polynucleotides may be altered by molecular manipulation of the coding sequence.

Due to the inherent degeneracy of the genetic code, other DNA sequences which encode substantially the same or a functionally equivalent amino acid sequence, may be used in the practice of the invention for the expression of the fusion polypeptides. Such DNA sequences include those which are capable of hybridizing to the coding sequences or their complements disclosed herein under low, moderate or high stringency conditions as described herein.

Altered nucleotide sequences which may be used in accordance with the invention include deletions, additions or substitutions of different nucleotide residues resulting in a sequence that encodes the same or a functionally equivalent gene product. The gene product itself may contain deletions, additions or substitutions of amino acid residues, which result in a silent change thus producing a functionally equivalent antigenic epitope. Such conservative amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine, histidine and arginine; amino acids with uncharged polar head groups having similar hydrophilicity values include the following: glycine, asparagine, glutamine, serine, threonine and tyrosine; and amino acids with nonpolar head groups include alanine, valine, isoleucine, leucine, phenylalanine, proline, methionine and tryptophan.

The nucleotide sequences of the invention may be engineered in order to alter the proteins, polypeptides, or fusion proteins coding sequence for a variety of ends, including but not limited to, alterations which modify processing and expression of the gene product. For example, mutations may be introduced using techniques which are well known in the art, *e.g.*, to insert or delete restriction sites, to alter glycosylation patterns, phosphorylation, to create and/or destroy translation, initiation, and/or termination sequences, or to create variations in coding regions, to facilitate further *in vitro* modification, etc. One of skill will recognize many ways of generating alterations in a given nucleic acid construct. Such well-known methods include, *e.g*., site-directed mutagenesis, PCR amplification using degenerate oligonucleotides, exposure of cells containing the nucleic acid to chemical mutagenic agents or radiation, chemical synthesis of a desired oligonucleotide (*e.g*., in conjunction with ligation and/or cloning to generate large nucleic acids) and other well-known techniques *(see, e.g*., Giliman et al. (1979) Gene 8:81-97; Hutchinson et al. (1978) J. Biol. Chem. 253:6551; Roberts et al. (1987) Nature 328: 731-734). Preferably, the manipulations do not destroy immunogenicity of the fusion polypeptides.

In one embodiment of the invention, the coding sequence of a proteins, polypeptides, or fusion proteins may be synthesized in whole or in part, using chemical methods well known in the art *(see, e.g.,* Caruthers et al. (1980) Nuc. Acids Res. Symp. Ser. 7:215-233; Crea et al. (1980) Nuc. Acids Res. 9(10):2331; Matteucci et al. (1980) Tetrahedron Letter 21:719 (1980); and Chow et al. (1981) Nuc. Acids Res. 9(12):2807-2817).

### B. Sequence Modifications

Variants of the proteins, polypeptides, or fusion proteins of the invention that retain the ability to stimulate an immune response may generally be identified by modifying the sequence in one or more of the aspects described above and assaying the resulting proteins, polypeptides, or fusion proteins for the ability to stimulate an immune response, *e.g*., a T cell response or an antibody response. For example, such assays may generally be performed by contacting T cells with the modified proteins, polypeptides, or fusion proteins and assaying the response. Naturally occurring variants of the individual polypeptide components of the proteins, polypeptides, or fusion proteins may also be isolated by, for example, screening an appropriate cDNA or genomic library with a DNA sequence encoding each individual polypeptide or a variant thereof.

The above-described sequence modifications may be introduced using standard recombinant techniques or by automated synthesis of the modified proteins, polypeptides, or fusion proteins. For example, mutations can be introduced at particular loci by synthesizing oligonucleotides containing a mutant sequence, flanked by restriction sites enabling ligation to fragments of the native sequence. Following ligation, the resulting reconstructed sequence encodes an analogue having the desired amino acid insertion, substitution, or deletion.

Alternatively, oligonucleotide-directed site-specific mutagenesis procedures can be used to provide a gene in which particular codons are altered according to the substitution, deletion, or insertion required. Exemplary methods of making the alterations set forth above are described by Walder et al. (1986) Gene 42:133; Bauer et al. (1985) Gene 37:73; Craik (1985) BioTechniques January:12-19; Smith et al. (1981) Genetic Engineering: Principles and Methods, Plenum Press; and U.S. Patent Nos. 4,518,584 and 4,737,462.

Mutations in nucleotide sequences constructed for expression of such proteins, polypeptides, or fusion proteins must, of course, preserve the reading frame of the coding sequences and preferably will not create complementary regions that could hybridize to produce secondary mRNA structures, such as loops or hairpins, which would adversely affect the translation of the mRNA. Although a mutation site may be predetermined, it is not necessary that the nature of the mutation *per se* be predetermined. For example, in order to select for optimum characteristics of mutants at a given site, random mutagenesis may be conducted at the target codon and the expressed protein mutants screened for the desired activity. Not all mutations in a nucleotide sequence which encodes a protein will be expressed in the final product. For example, nucleotide substitutions may be made to enhance expression, primarily to avoid secondary structure loops in the transcribed mRNA (*see, e.g.,* European Patent Application 75,444A), or to provide codons that are more readily translated by the selected host, such as the well-known *E. coli* preference codons for *E. coli* expression.

### C. Expression Vectors

The proteins, polypeptides, or fusion proteins, and variants thereof, of the present invention, are preferably produced by recombinant DNA methods. Such methods include, for example, inserting a DNA sequence encoding a cancer antigen protein, polypeptide, or fusion protein into the recombinant expression vector described above and expressing the DNA sequence in a recombinant microbial, mammalian, fungal or insect cell expression system under conditions promoting expression and, preferably, secretion of the proteins, polypeptides, or fusion proteins. DNA sequences encoding the proteins, polypeptides, or fusion proteins provided by this invention can be assembled from cDNA fragments and short oligonucleotide linkers, or from a series of oligonucleotides, to provide a synthetic gene which is capable of being inserted in a recombinant expression vector and expressed in a recombinant transcriptional unit.

Recombinant expression vectors contain a DNA sequence encoding a protein operably linked to suitable transcriptional or translational regulatory elements derived from mammalian, fungal, microbial, viral or insect genes. Such regulatory elements include a transcriptional promoter, an optional operator sequence to control transcription, a sequence encoding suitable mRNA ribosomal binding sites, and sequences which control the termination of transcription and translation. An origin of replication and a selectable marker to facilitate recognition of transformants may additionally be incorporated.

DNA regions are "operably linked" when they are functionally related to each other. For example, DNA for a signal peptide (secretory leader) is operably linked to DNA for a polypeptide if it is expressed as a precursor which participates in the secretion of the polypeptide; a promoter is "operably linked" to a coding sequence if it controls the transcription of the sequence; or a ribosome binding site is "operably linked" to a coding sequence if it is positioned so as to permit translation. Generally, "operably linked" means contiguous and, in the case of secretory leaders, in reading frame. DNA sequences encoding proteins, polypeptides, or fusion proteins which are to be expressed in a microorganism will preferably contain no introns that could prematurely terminate transcription of DNA into mRNA.

Expression vectors for bacterial use may comprise a selectable marker and a bacterial origin of replication derived from commercially available plasmids comprising genetic elements of the well known cloning vector pBR322 (ATCC 37017). Such commercial vectors include, *e.g*., pKK223-3 (Pharmacia Fine Chemicals, Uppsala, Sweden), pGEM1 (Promega Biotec, Madison, WI), pET28b (Novagen) and pPDM (a modified pET28b, Corixa). These pBR322 "backbone" sections are combined with an appropriate promoter and the structural sequence to be expressed. *E. coli* is typically transformed using derivatives of pBR322, a plasmid derived from an *E. coli* species (Bolivar et al. (1977) Gene 2:95)*.* pBR322 contains genes for ampicillin and tetracycline resistance and thus provides simple means for identifying transformed cells.

Preferred yeast vectors can be assembled using DNA sequences from pBR322 for selection and replication in *E*. *coli* (Amp^{r} gene and origin of replication) and yeast DNA. The yeast α-factor leader, which directs secretion of heterologous proteins, can be inserted between the promoter and the structural gene to be expressed *(see, e.g.,* Kurjan et al. (1982) Cell 30:933; and Bitter et al. (1984) Proc. Natl. Acad. Sci. USA 81:5330. The leader sequence may be modified to contain, near its 3' end, one or more useful restriction sites to facilitate fusion of the leader sequence to foreign genes.

In addition to the transcriptional and translational control sequences, some expression systems have markers that provide gene amplification, such as neomycin, thymidine kinase, hygromycin B phosphotransferase, and dihydrofolate reductase.

A useful cell line that allows for episomal replication of expression vectors is CV-1/EBNA (ATCC CRL 10478). The CV-1/EBNA cell line was derived by transfection of the CV-1 cell line with a gene encoding Epstein-Barr virus nuclear antigen-I (EBNA-1) and constitutively expresses EBNA-1 driven from human CMV immediate-early enhancer/promoter.

Preferred vectors for expression in mammalian cultured cells include pFLAGCMV-1 (Kodak), pcDNA3.1/hyg (Invitrogen), pEE14-GS (CellTech), pBIB and VR1012 (Hartikka et al. (1996) Hum. Gene Ther. 7(10):1205-1217).

Any of the well known procedures for introducing foreign nucleotide sequences into host cells may be used to introduce the expression vector. These include the use of reagents such as Superfect (Qiagen), liposomes, calcium phosphate transfection, polybrene, protoplast fusion, electroporation, microinjection, plasmid vectors, viral vectors, biolistic particle acceleration (the gene gun), or any of the other well known methods for introducing cloned genomic DNA, cDNA, synthetic DNA or other foreign genetic material into a host cell *(see, e.g*., Sambrook *et al., supra*).

### D. Host Cells

Transformed host cells are cells which have been transformed or transfected with expression vectors constructed using recombinant DNA techniques and which contain sequences encoding proteins, polypeptides, or fusion proteins of interest. Transformed host cells may express the desired proteins, polypeptides, or fusion proteins of interest, but host cells transformed for purposes of cloning or amplifying a DNA do not need to express the proteins, polypeptides, or fusion proteins of interest. Expressed proteins, polypeptides, or fusion proteins will preferably be secreted into the culture medium or supernatant, depending on the DNA selected. One skilled in the art will appreciate that if proteins, polypeptides, or fusion proteins are secreted into the culture supernatant, then they are also soluble in the culture supernatant.

Any of the well known procedures for introducing foreign nucleotide sequences into host cells may be used to introduce the expression vector. These include the use of reagents such as Superfect (Qiagen), liposomes, calcium phosphate transfection, polybrene, protoplast fusion, electroporation, microinjection, plasmid vectors, viral vectors, biolistic particle acceleration (the gene gun), or any of the other well known methods for introducing cloned genomic DNA, cDNA, synthetic DNA or other foreign genetic material into a host cell *(see, e.g*., Sambrook *et al., supra*).

Suitable host cells for expression of recombinant proteins include prokaryotes, yeast or higher eukaryotic cells under the control of appropriate promoters. Examples of suitable mammalian host cell lines include the COS-7 lines of monkey kidney cells, described by Gluzman (1981) Cell 23:175, and other cell lines capable of expressing an appropriate vector including, *e.g*., CV-1/EBNA (ATCC CRL 10478), L cells, C127, 3T3, Chinese hamster ovary (CHO), COS, NS-1, HeLa, Human embryonic Kidney Fibroblasts (HEK 293), BHK and HEK293 cell lines. Mammalian expression vectors may comprise nontranscribed elements (*e.g*., an origin of replication, a suitable promoter and/or an enhancer linked to the gene to be expressed, and other 5' or 3' flanking nontranscribed sequences) and 5' or 3' nontranslated sequences (*e.g*., necessary ribosome binding sites, a polyadenylation site, splice donor and acceptor sites, and transcriptional termination sequences). Preferred mammalian expression systems are the Chinese hamster ovary (CHO), the HEK293 and the BHK cell lines. Recombinant CHO-expressed protein, polypeptide, or fusion protein are secreted into the cell supernatant as a glycosylated protein.

Prokaryotes include gram negative or gram positive organisms, for example *E. coli* or *Bacilli.* Higher eukaryotic cells include established cell lines of insect or mammalian origin as described below. Cell-free translation systems could also be used to produce proteins, polypeptides, or fusion proteins using RNAs derived from DNA constructs. Appropriate cloning and expression vectors for use with bacterial, fungal, yeast, and mammalian cellular hosts are described, for example, by Pouwels et al., Cloning Vectors: A Laboratory Manual, Elsevier, NY (1985).

Prokaryotic expression hosts may be used for expression of proteins, polypeptides, or fusion proteins that do not require extensive proteolytic and disulfide processing. Prokaryotic expression vectors generally comprise one or more phenotypic selectable markers, *e.g*., a gene encoding a protein conferring antibiotic resistance or supplying an autotrophic requirement, and an origin of replication recognized by the host to ensure amplification within the host. Suitable prokaryotic hosts for transformation include *E*. *coli* (*e.g*., BL21 (DE3) CodonPlus *E. coli*), *Bacillus subtilis, Salmonella typhimurium,* and various species within the genera *Pseudomonas, Streptomyces,* and *Staphylococcus,* although other hosts may also be used.

Insect (*e.g., Spodoptera* or *Trichoplusia*) cell culture systems can also be used to express recombinant polypeptides. Baculovirus systems for production of heterologous polypeptides in insect cells are reviewed, for example, by Luckow et al. (1988) BioTechnology 6:47.

### E. Purification of the Proteins of Interest

It will be understood by one of skill in the art that purified proteins, polypeptides, or fusion proteins of interest may be prepared by culturing suitable host/vector systems to express the recombinant translation products of the DNAs of the present invention, which are then purified from culture media or cell extracts. For example, supernatants from systems which secrete recombinant polypeptides into culture media may be first concentrated using a commercially available protein concentration filter, such as, *e.g.*, an Amicon or Millipore Pellicon ultrafiltration unit. Following the concentration step, the concentrate may be applied to a suitable purification matrix. For example, a suitable affinity matrix may comprise a counter structure protein (*i.e.,* a protein to which a protein, polypeptide, or fusion protein of interest binds in a specific interaction based on structure) or lectin or antibody molecule bound to a suitable support.

Alternatively, an anion exchange resin can be used, for example, a matrix or substrate having pendant diethylaminoethyl (DEAE) groups. The matrices can be acrylamide, agarose, dextran, cellulose, polystyrene, sepharose or other types commonly used in protein purification. Alternatively, a cation exchange step can be used. Suitable cation exchangers include various insoluble matrices comprising sulfopropyl or carboxy-methyl groups, preferably sulfopropyl groups. Gel filtration chromatography also provides a means of purifying proteins, polypeptides, or fusion proteins. The proteins, polypeptides, or fusion proteins of the invention are preferably purified by anion exchange chromatography using, *e.g*., monoQ columns or Q sepharose High Performance chromatography.

Affinity chromatography is another preferred method of purifying proteins, polypeptides, or fusion proteins. For example, monoclonal antibodies against the proteins, polypeptides, or fusion proteins may be useful in affinity chromatography purification, by using methods that are well-known in the art.

Finally, one or more reverse-phase high performance liquid chromatography (RP-HPLC) steps using hydrophobic RP-HPLC media (*e.g*., silica gel having pendant methyl or other aliphatic groups) may be used to further purify proteins, polypeptides, or fusion proteins of interest. Some or all of the foregoing purification steps, in various combinations, can also be used to provide a homogeneous recombinant protein or polypeptide.

Recombinant proteins, polypeptides, or fusion proteins produced in bacterial culture may be purified by initial extraction from cell pellets, followed by one or more concentration, salting-out, aqueous ion exchange or size exclusion chromatography steps. High performance liquid chromatography (HPLC) may be used for final purification steps. Microbial cells used in expression of recombinant proteins, polypeptides, or fusion proteins can be disrupted by any convenient method, including freeze-thaw cycling, sonication, mechanical disruption, or use of cell lysing agents.

Fermentation of yeast cells which express proteins, polypeptides, or fusion proteins as a secreted protein greatly simplifies purification. The secreted recombinant proteins resulting from a large-scale fermentation can be purified by methods analogous to those disclosed by Urdal et al. (1984) J. Chromatog. 296:171. This reference describes two sequential, reverse-phase HPLC steps for purification of recombinant human GM-CSF on a preparative HPLC column.

Preparations of proteins, polypeptides, or fusion proteins synthesized in recombinant cultures may contain non- proteins, polypeptides, or fusion proteins cell components, including proteins, in amounts and of a character which depend upon the purification steps taken to recover the proteins, polypeptides, or fusion proteins from the culture. These components are ordinarily of yeast, prokaryotic or non-human eukaryotic origin. Such preparations are typically free of other proteins which may be normally associated with the protein of interest as it is found in nature in its species of origin.

Automated synthesis provides an alternate method for preparing proteins and polypeptides of this invention. For example, any of the commercially available solid-phase techniques may be used, such as, *e.g*., the Merrifield solid phase synthesis method, in which amino acids are sequentially added to a growing amino acid chain (*see,* Merrifield (1963) J. Am. Chem. Soc. 85:2149-2146). Equipment for automated synthesis of polypeptides is commercially available from suppliers such as Applied Biosystems, Inc. (Foster City, CA), and may generally be operated according to the manufacturer's instructions.

### V. Compositions of the Invention

The DNA vectors of the invention and the polypeptides encoded by the heterologous nucleic acid sequences therein can conveniently be incorporated into pharmaceutical compositions or immunogenic compositions (*i.e.*, vaccines). Pharmaceutical compositions comprise the vectors and a physiologically acceptable carrier or excipient. Vaccines may comprise one or more such compounds or cells and an immunostimulant, such as an adjuvant or a liposome (into which the compound is incorporated). An immunostimulant may be any substance that enhances or potentiates an immune response (antibody- and/or cell-mediated) to an exogenous antigen. Examples of immunostimulants include adjuvants, biodegradable microspheres (*e.g*., polylactic galactide) and liposomes (into which the compound is incorporated) (U. S. Patent No. 4,235,877). Vaccine preparation is generally described in, for example, Powell and Newman (1995). Pharmaceutical compositions and vaccines within the scope of the present invention may also contain other compounds, which may be biologically active or inactive. For example, one or more immunogenic portions of other tumor antigens may be present, either incorporated into a fusion peptide or as a separate compound, within the composition or vaccine.

Within certain embodiments, pharmaceutical compositions and vaccines are designed to elicit T cell responses specific for a hematological malignancy related peptide in a patient, such as a human. In general, T cell responses may be favored through the use of heterologous nucleic acids encoding relatively short peptides (*e.g*., comprising less than 23 consecutive amino acid residues of a native hematological malignancy related peptide, preferably 4-16 consecutive residues, more preferably 8-16 consecutive residues and still more preferably 8-10 consecutive residues). Alternatively, or in addition, a vaccine may comprise an immunostimulant that preferentially enhances a T cell response. In other words, the immunostimulant may enhance the level of a T cell response to a hematological malignancy related peptide by an amount that is proportionally greater than the amount by which an antibody response is enhanced. For example, when compared to a standard oil based adjuvant, such as CFA, an immunostimulant that preferentially enhances a T cell response may enhance a proliferative T cell response by at least two fold, a lytic response by at least 10%, and/or T cell activation by at least two fold compared to hematological malignancy related-negative control cell lines, while not detectably enhancing an antibody response. The amount by which a T cell or antibody response to a polypeptide encoded by the heterologous nucleic acid is enhanced may generally be determined using any representative technique known in the art, such as the techniques provided herein.

A pharmaceutical composition or vaccine may contain nucleic acid sequences encoding one or more of the cancer antigens as described above, such that a polypeptide is generated *in situ.* As noted above, the DNA may be present within any of a variety of delivery systems known to those of ordinary skill in the art, including nucleic acid expression systems, bacterial and viral expression systems and mammalian expression systems. Numerous gene delivery techniques are well known in the art (Rolland, 1998, and references cited therein). Appropriate nucleic acid expression systems contain the necessary DNA, cDNA or RNA sequences for expression in the patient (such as a suitable promoter and terminating signal). Bacterial delivery systems involve the administration of a bacterium (such as *Bacillus-Calmette-Guerrin*) that expresses an immunogenic portion of the peptide on its cell surface or secretes such an epitope. In a preferred embodiment, the DNA may be introduced using a viral expression system (*e.g*., vaccinia or other pox virus, retrovirus, or adenovirus), which may involve the use of a non-pathogenic (defective), replication competent virus (Fisher-Hoch *et al.,* 1989; Flexner *et al.,* 1989; Flexner *et al.,* 1990; U. S. Patent No. 4,603,112, U. S. Patent No. 4,769,330, U. S. Patent No. 5,017,487; Intl. Pat. Appl. Publ, No. WO 89/01973; U. S. Patent No. 4,777,127; Great Britain Patent No. GB 2,200,651; European Patent No. EP 0,345,242; Intl. Pat. Appl. Publ. No. WO 91/02805; Berkner, 1988; Rosenfeld *et al.,* 1991; Kolls *et al.,* 1994; Kass-Eisler *et al.,* 1993; Guzman *et al.,* 1993a; and Guzman *et al.,* 1993). Techniques for incorporating DNA into such expression systems are well known to those of ordinary skill in the art. It will be apparent that a vaccine may comprise both a polynucleotide and a peptide component. Such vaccines may provide for an enhanced immune response.

It will be apparent to those of ordinary skill in the art having the benefit of the present teachings that a vaccine may contain pharmaceutically acceptable carriers. The phrases "pharmaceutically or pharmacologically acceptable" refer to molecular entities and compositions that do not produce an adverse, allergic or other significant untoward reaction when administered to an animal, or a human, as appropriate. As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. For human administration, preparations should meet sterility, pyrogenicity, and general safety and purity standards as required by the Food and Drug Administration Office of Biologics standards. Supplementary active ingredients can also be incorporated into the compositions.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g*., polylactate polyglycolate) may also be employed as carriers for the pharmaceutical compositions of this invention. Suitable biodegradable microspheres are disclosed, for example, in U. S. Patent Nos. 4,897,268; 5,075,109; 5,928,647; 5,811,128; 5,820,883; 5,853,763; 5,814,344 and 5,942,252. For certain topical applications, formulation as a cream or lotion, using well-known components, is preferred.

Such compositions may also comprise buffers (*e.g*., neutral buffered saline or phosphate buffered saline), carbohydrates (*e.g*., glucose, mannose, sucrose or dextrans), mannitol, proteins, peptides or amino acids such as glycine, antioxidants, bacteriostats, chelating agents such as EDTA or glutathione, adjuvants (*e.g*., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate, or formulated with one or more liposomes, microspheres, nanoparticles, or micronized delivery systems using well-known technology.

Any of a variety of immunostimulants, such as adjuvants, may be employed in the preparation of vaccine compositions of this invention. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, alum-based adjuvants (*e.g*., Alhydrogel, Rehydragel, aluminum phosphate, Algammulin, aluminum hydroxide); oil based adjuvants (Freund's Incomplete Adjuvant and Complete Adjuvant (Difco Laboratories, Detroit, MI), Specol, RIBI, TiterMax, Montanide ISA50 or Seppic MONTANIDE ISA 720); nonionic block copolymer-based adjuvants, cytokines (*e.g*., GM-CSF or Flat3-ligand); Merck Adjuvant 65 (Merck and Company, Inc., Rahway, NJ); AS-2 (SmithKline Beecham, Philadelphia, PA); salts of calcium, iron or zinc; an insoluble suspension of acylated tyrosine; acylated sugars; cationically or anionically derivatized polysaccharides; polyphosphazenes; biodegradable microspheres; monophosphoryl lipid A and Quil A. Cytokines, such as GM-CSF or interleukin-2, -7, or - 12, may also be used as adjuvants.

Hemocyanins and hemoerythrins may also be used in the invention. The use of hemocyanin from keyhole limpet (KLH) is particularly preferred, although other molluscan and arthropod hemocyanins and hemoerythrins may be employed. Various polysaccharide adjuvants may also be used. Polyamine varieties of polysaccharides are particularly preferred, such as chitin and chitosan, including deacetylated chitin.

A further preferred group of adjuvants are the muramyl dipeptide (MDP, N-acetylmuramyl-L-alanyl-D-isoglutamine) group of bacterial peptidoglycans. Derivatives of muramyl dipeptide, such as the amino acid derivative threonyl-MDP, and the fatty acid derivative MTPPE, are also contemplated.

U. S. Patent No. 4,950,645 describes a lipophilic disaccharide-tripeptide derivative of muramyl dipeptide that is proposed for use in artificial liposomes formed from phosphatidyl choline and phosphatidyl glycerol. It is said to be effective in activating human monocytes and destroying tumor cells, but is non-toxic in generally high doses. The compounds of U. S. Patent No. 4,950,645, and Intl. Pat. Appl. Publ. No. WO 91/16347 are also proposed for use in achieving particular aspects of the present invention.

BCG and BCG-cell wall skeleton (CWS) may also be used as adjuvants in the invention, with or without trehalose dimycolate. Trehalose dimycolate may be used itself. Azuma *et al.* (1988) show that trehalose dimycolate administration correlates with augmented resistance to influenza virus infection in mice. Trehalose dimycolate may be prepared as described in U. S. Patent No. 4,579,945.

Amphipathic and surface-active agents, *e.g.*, saponin and derivatives such as QS21 (Cambridge Biotech), form yet another group of preferred adjuvants for use with the immunogens of the present invention. Nonionic block copolymer surfactants (Rabinovich *et al.,* 1994; Hunter *et al.,* 1991) may also be employed. Oligonucleotides, as described by Yamamoto *et al.* (1988) are another useful group of adjuvants. Quil A and lentinen are also preferred adjuvants.

Superantigens are also contemplated for use as adjuvants in the present invention. "Superantigens" are generally bacterial products that stimulate a greater proportion of T lymphocytes than peptide antigens without a requirement for antigen processing (Mooney *et. al.,* 1994). Superantigens include *Staphylococcus* exoproteins, such as the α, β, γ and δ enterotoxins from *S*. *aureus* and *S. epidermidis,* and the α, β, γ and δ *E. coli* exotoxins.

Common *Staphylococcus* enterotoxins are known as staphylococcal enterotoxin A (SEA) and staphylococcal enterotoxin B (SEB), with enterotoxins through E (SEE) being described (Rott *et. al.,* 1992). *Streptococcus pyogenes* B (SEB), *Clostridium perfringens* enterotoxin (Bowness *et. al.,* 1992), cytoplasmic membrane-associated protein (CAP) from *S. pyogenes* (Sato *et. al.,* 1994) and toxic shock syndrome toxin-1 (TSST-1) from *S*. *aureus* (Schwab *et. al.,* 1993) are further useful superantigens.

One group of adjuvants particularly preferred for use in the invention are the detoxified endotoxins, such as the refined detoxified endotoxin of U. S. Patent No. 4,866,034. These refined detoxified endotoxins are effective in producing adjuvant responses in mammals.

The detoxified endotoxins may be combined with other adjuvants. Combination of detoxified endotoxins, with trehalose dimycolate is contemplated, as described in U. S. Patent No. 4,435,386. Combinations of detoxified endotoxins with trehalose dimycolate and endotoxic glycolipids is also contemplated (U. S. Patent No. 4,505,899), as is combination of detoxified endotoxins with cell wall skeleton (CWS) or CWS and trehalose dimycolate, as described in U. S. Patent Nos. 4,436,727, 4,436,728 and 4,505,900. Combinations of just CWS and trehalose dimycolate, without detoxified endotoxins are also envisioned to be useful, as described in U. S. Patent No. 4,520,019.

MPL is currently one preferred immunopotentiating agent for use herein. References that concern the uses of MPL include Tomai *et al.* (1987), Chen *et al.* (1991) and Garg and Subbarao (1992), that each concern certain roles of MPL in the reactions of aging mice; Elliott *et al.* (1991), that concerns the D-galactosamine loaded mouse and its enhanced sensitivity to lipopolysaccharide and MPL; Chase *et al.* (1986), that relates to bacterial infections; and Masihi *et al.* (1988), that describes the effects of MPL and endotoxin on resistance of mice to *Toxoplasma gondii.* Fitzgerald (1991) also reported on the use of MPL to up-regulate the immunogenicty of a syphilis vaccine and to confer significant protection against challenge infection in rabbits.

Baker *et al.* (1992) further analyzed the structural features that influence the ability of lipid A and its analogs to abolish expression of suppressor T cell activity. They reported that decreasing the number of phosphate groups in lipid A from two to one (*i.e*., creating monophosphoryl lipid A, MPL) as well as decreasing the fatty acyl content, primarily by removing the residue at the 3 position, resulted in a progressive reduction in toxicity; however, these structural modifications did not influence its ability to abolish the expression of Ts function (Baker *et al.,* 1992). These types of MPL are ideal for use in the present invention.

In a generally related line of work, Tanamoto *et al.* (1994a;b; 1995) described the dissociation of endotoxic activities in a chemically synthesized Lipid A precursor after acetylation or succinylation. Thus, compounds such as "acetyl 406" and "succinyl 516" (Tanamoto *et al.,* 1994a;b; 1995) are also contemplated for use in the invention.

Synthetic MPLs form a particularly preferred group of adjuvants. For example, Brade *et al.* (1993) described an artificial glycoconjugate containing the bisphosphorylated glucosamine disaccharide backbone of lipid A that binds to anti-Lipid A MAbs. This is one candidate for use in certain aspects of the invention.

The MPL derivatives described in U. S. Patent No. 4,987,237 are particularly contemplated for use in the present invention. U. S. Patent No. 4,987,237 describes MPL derivatives that contain one or more free groups, such as amines, on a side chain attached to the primary hydroxyl groups of the monophosphoryl lipid A nucleus through an ester group. The derivatives provide a convenient method for coupling the lipid A through coupling agents to various biologically active materials. The immunostimulant properties of lipid A are maintained. All MPL derivatives in accordance with U. S. Patent No. 4,987,237 are envisioned for use in the MPL adjuvant-incorporated cells of this invention.

Various adjuvants, even those that are not commonly used in humans, may still be employed in animals, where, for example, one desires to raise antibodies or to subsequently obtain activated T cells. The toxicity or other adverse effects that may result from either the adjuvant or the cells, *e.g.*, as may occur using non-irradiated tumor cells, is irrelevant in such circumstances.

Within the vaccines provided herein, the adjuvant composition is preferably designed to induce an immune-response predominantly of the Th1 type. High levels of Th1-type cytokines (*e.g*., IFN-γ, TNFα, IL-2 and IL-12) tend to favor the induction of cell-mediated immune responses to an administered antigen. In contrast, high levels of Th2-type cytokines (*e.g*., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines see *e.g*., Mosmann and Coffman (1989).

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Corixa Corporation (Seattle, WA; see *e.g*., U. S. Patent Nos. 4,436,727; 4,877,611; 4,866,034 and 4,912,094, each of which is specifically incorporated herein by reference in its entirety). CpG-containing oligonucleotides (in which the CpG dinucleotide is unmethylated) also induce a predominantly The response. Such oligonucleotides are well known and are described, for example, in Intl. Pat. Appl. Publ. No. WO 96/02555 and Intl. Pat. Appl. Publ. No. WO 99/33488. Immunostimulatory DNA sequences are also described, for example, by Sato *et al.* (1996). Another preferred adjuvant is a saponin, preferably QS21 (Aquila Biopharmaceuticals Inc., Framingham, MA), which may be used alone or in combination with other adjuvants. For example, an enhanced system involves the combination of a monophosphoryl lipid A and saponin derivative, such as the combination of QS21 and 3D-MPL (see *e.g*., Intl. Pat. Appl. Publ. No. WO 94/00153), or a less reactogenic composition where the QS21 is quenched with cholesterol (see *e.g*., Intl. Pat. Appl. Publ. No.
WO 96/33739). Other preferred formulations comprise an oil-in-water emulsion and tocopherol. A particularly potent adjuvant formulation involving QS21, 3D-MPL and tocopherol in an oil-in-water emulsion has also been described (see *e.g*., Intl. Pat. Appl. Publ. No. WO 95/17210).

Other preferred adjuvants include Montanide ISA 720 (Seppic), SAF (Chiron), ISCOMS (CSL), MF-59 (Chiron), Detox (Corixa Corporation), RC-529 (Corixa Corporation) and aminoalkyl glucosaminide 4-phosphates (AGPs).

Any vaccine provided herein may be prepared using well-known methods that result in a combination of one or more antigens, one or more immunostimulants or adjuvants and one or more suitable carriers, excipients, or pharmaceutically acceptable buffers. The compositions described herein may be administered as part of a sustained release formulation (*i.e.,* a formulation such as a capsule, sponge or gel [composed of polysaccharides, for example] that effects a slow release of compound following administration). Such formulations may generally be prepared using well-known technology (Coombes *et al.,* 1996) and administered by, for example, oral, rectal or subcutaneous implantation, or by implantation at the desired target site. Sustained-release formulations may contain a peptide, polynucleotide or antibody dispersed in a carrier matrix and/or contained within a reservoir surrounded by a rate-controlling membrane.

Carriers for use within such formulations are preferably biocompatible, and may also be biodegradable; preferably the formulation provides a relatively constant level of active component release. Such carriers include microparticles of poly(lactide-co-glycolide), as well as polyacrylate, latex, starch, cellulose and dextran. Other delayed-release carriers include supramolecular biovectors, which comprise a non-liquid hydrophilic core (*e.g*., a cross-linked polysaccharide or oligosaccharide) and, optionally, an external layer comprising an amphiphilic compound, such as a phospholipid (U. S. Patent No. 5,151,254; Intl. Pat. Appl. Publ. No. WO 94/20078; Intl. Pat. Appl. Publ. No. WO/94/23701; and Intl. Pat. Appl. Publ. No. WO 96/06638). The amount of active compound contained within a sustained release formulation depends upon the site of implantation, the rate and expected duration of release and the nature of the condition to be treated or prevented.

Combined therapeutics is also contemplated, and the same type of underlying pharmaceutical compositions may be employed for both single and combined medicaments. Vaccines and pharmaceutical compositions may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a vaccine or pharmaceutical composition may be stored in a freeze-dried condition requiring only the addition of a sterile liquid carrier immediately prior to use.

### VI. Administration of Pharmaceutical Compositions Comprising the Expression Vector

In some embodiments, the pharmaceutical compositions comprising the expression vectors disclosed herein may be delivered to an individual with cancer, typically a mammal *e.g*., a human, a chimpanzee, a canine, or a feline.

### A. Oral Delivery

In certain applications, the pharmaceutical compositions comprising the expression vectors disclosed herein may be delivered *via* oral administration to the individual. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (Mathiowitz *et al.,* 1997; Hwang *et al.,* 1998; U. S. Patent 5,641,515; U. S. Patent 5,580,579 and U. S. Patent 5,792,451). The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

Typically, these formulations may contain at least about 0.1 % of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

### B. Injectable Delivery

In certain circumstances it will be desirable to deliver the pharmaceutical compositions disclosed herein parenterally, intravenously, intramuscularly, or even intraperitoneally as described in U. S. Patent 5,543,158; U. S. Patent 5,641,515 and U. S. Patent 5,399,363. Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions (U. S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e.g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion (*see, e.g.,* Remington's Pharmaceutical Sciences, 15th Edition, pp. 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, and the general safety and purity standards as required by FDA Office of Biologies standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

### C. Nasal Delivery

In certain embodiments, the pharmaceutical compositions may be delivered by intranasal sprays, inhalation, and/or other aerosol delivery vehicles. Methods for delivering genes, nucleic acids, and peptide compositions directly to the lungs via nasal aerosol sprays has been described *e.g.*, in U. S. Patent 5,756,353 and U. S. Patent 5,804,212. Likewise, the delivery of drugs using intranasal microparticle resins (Takenaga *et al.,* 1998) and lysophosphatidyl-glycerol compounds (U. S. Patent 5,725,871) are also well-known in the pharmaceutical arts. Likewise, transmucosal drug delivery in the form of a polytetrafluoroetheylene support matrix is described in U. S. Patent 5,780,045.

### D. Liposome-, Nanocapsule-, And Microparticle-Mediated Delivery

In certain embodiments, the inventors contemplate the use of liposomes, nanocapsules, microparticles, microspheres, lipid particles, vesicles, and the like, for the introduction of the compositions of the present invention into suitable host cells. In particular, the compositions of the present invention may be formulated for delivery either encapsulated in a lipid particle, a liposome, a vesicle, a nanosphere, or a nanoparticle or the like.

Such formulations may be preferred for the introduction of pharmaceutically-acceptable formulations of the nucleic acids or constructs disclosed herein. The formation and use of liposomes is generally known to those of skill in the art (see for example, Couvreur *et al.,* 1977; Couvreur, 1988; Lasic, 1998; which describes the use of liposomes and nanocapsules in the targeted antibiotic therapy for intracellular bacterial infections and diseases). Recently, liposomes were developed with improved serum stability and circulation half-times (Gabizon & Papahadjopoulos, 1988; Allen and Choun, 1987; U. S. Patent 5,741,516). Further, various methods of liposome and liposome like preparations as potential drug carriers have been reviewed (Takakura, 1998; Chandran *et al.,* 1997; Margalit, 1995; U. S. Patent 5,567,434; U. S. Patent 5,552,157; U. S. Patent 5,565,213; U. S. Patent 5,738,868 and U. S. Patent 5,795,587).

Liposomes have been used successfully with a number of cell types that are normally resistant to transfection by other procedures including T cell suspensions, primary hepatocyte cultures and PC 12 cells (Renneisen *et al.,* 1990; Muller *et al.,* 1990). In addition, liposomes are free of the DNA length constraints that are typical of viral-based delivery systems. Liposomes have been used effectively to introduce genes, drugs (Heath & Martin, 1986; Heath *et al.,* 1986; Balazsovits *et al.,* 1989; Fresta & Puglisi, 1996), radiotherapeutic agents (Pikul *et al.,* 1987), enzymes (Imaizumi *et al.,* 1990a; Imaizumi *et al.,* 1990b), viruses (Faller & Baltimore, 1984), transcription factors and allosteric effectors (Nicolau & Gersonde, 1979) into a variety of cultured cell lines and animals. In addition, several successful clinical trails examining the effectiveness of liposome-mediated drug delivery have been completed (Lopez-Berestein *et al.,* 1985a; 1985b; Coune, 1988; Sculier *et al.,* 1988). Furthermore, several studies suggest that the use of liposomes is not associated with autoimmune responses, toxicity or gonadal localization after systemic delivery (Mori & Fukatsu, 1992).

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 A, containing an aqueous solution in the core.

Liposomes bear resemblance to cellular membranes and are contemplated for use in connection with the present invention as carriers for the peptide compositions. They are widely suitable as both water- and lipid-soluble substances can be entrapped, i.e. in the aqueous spaces and within the bilayer itself, respectively. It is possible that the drug-bearing liposomes may even be employed for site-specific delivery of active agents by selectively modifying the liposomal formulation.

In addition to the teachings of Couvreur *et al.* (1977; 1988), the following information may be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs at a characteristic phase-transition temperature and results in an increase in permeability to ions, sugars and drugs.

In addition to temperature, exposure to proteins can alter the permeability of liposomes. Certain soluble proteins, such as cytochrome c, bind, deform and penetrate the bilayer, thereby causing changes in permeability. Cholesterol inhibits this penetration of proteins, apparently by packing the phospholipids more tightly. It is contemplated that the most useful liposome formations for antibiotic and inhibitor delivery will contain cholesterol.

The ability to trap solutes varies between different types of liposomes. For example, MLVs are moderately efficient at trapping solutes, but SUVs are extremely inefficient. SUVs offer the advantage of homogeneity and reproducibility in size distribution, however, and a compromise between size and trapping efficiency is offered by large unilamellar vesicles (LUVs). These are prepared by ether evaporation and are three to four times more efficient at solute entrapment than MLVs.

In addition to liposome characteristics, an important determinant in entrapping compounds is the physicochemical properties of the compound itself. Polar compounds are trapped in the aqueous spaces and nonpolar compounds bind to the lipid bilayer of the vesicle. Polar compounds are released through permeation or when the bilayer is broken, but nonpolar compounds remain affiliated with the bilayer unless it is disrupted by temperature or exposure to lipoproteins. Both types show maximum efflux rates at the phase transition temperature.

Liposomes interact with cells *via* four different mechanisms: endocytosis by phagocytic cells of the reticuloendothelial system such as macrophages and neutrophils; adsorption to the cell surface, either by nonspecific weak hydrophobic or electrostatic forces, or by specific interactions with cell-surface components; fusion with the plasma cell membrane by insertion of the lipid bilayer of the liposome into the plasma membrane, with simultaneous release of liposomal contents into the cytoplasm; and by transfer of liposomal lipids to cellular or subcellular membranes, or vice versa, without any association of the liposome contents. It often is difficult to determine which mechanism is operative and more than one may operate at the same time.

The fate and disposition of intravenously injected liposomes depend on their physical properties, such as size, fluidity, and surface charge. They may persist in tissues for h or days, depending on their composition, and half lives in the blood range from min to several h. Larger liposomes, such as MLVs and LUVs, are taken up rapidly by phagocytic cells of the reticuloendothelial system, but physiology of the circulatory system restrains the exit of such large species at most sites. They can exit only in places where large openings or pores exist in the capillary endothelium, such as the sinusoids of the liver or spleen. Thus, these organs are the predominate site of uptake. On the other hand; SUVs show a broader tissue distribution but still are sequestered highly in the liver and spleen. In general, this *in vivo* behavior limits the potential targeting of liposomes to only those organs and tissues accessible to their large size. These include the blood, liver, spleen, bone marrow, and lymphoid organs.

Targeting is generally not a limitation in terms of the present invention. However, should specific targeting be desired, methods are available for this to be accomplished. Antibodies may be used to bind to the liposome surface and to direct the antibody and its drug contents to specific antigenic receptors located on a particular cell-type surface. Carbohydrate determinants (glycoprotein or glycolipid cell-surface components that play a role in cell-cell recognition, interaction and adhesion) may also be used as recognition sites as they have potential in directing liposomes to particular cell types. Mostly, it is contemplated that intravenous injection of liposomal preparations would be used, but other routes of administration are also conceivable.

Alternatively, the invention provides for pharmaceutically-acceptable nanocapsule formulations of the compositions of the present invention. Nanocapsules can generally entrap compounds in a stable and reproducible way (Henry-Michelland *et al.,* 1987; Quintanar-Guerrero *et al.,* 1998; Douglas *et al.,* 1987). To avoid side effects due to intracellular polymeric overloading, such ultrafine particles (sized around 0.1 µm) should be designed using polymers able to be degraded *in vivo.* Biodegradable polyalkyl-cyanoacrylate nanoparticles that meet these requirements are contemplated for use in the present invention. Such particles may be are easily made, as described (Couvreur *et al.,* 1980; 1988; zur Muhlen *et al.,* 1998; Zambaux *et al.* 1998; Pinto-Alphandry *et al.,* 1995 and U. S. Patent 5,145,684).

### VII. Binding Agents

The present invention further provides agents, such as antibodies and antigen-binding fragments thereof, that specifically bind to a cancer antigen or oncogene encoded by the heterologous nucleic acid in the expression vector of the invention. As used herein, an antibody, or antigen-binding fragment thereof, is said to "specifically bind" to a protein if it reacts at a detectable level, *i.e.,* at least two fold over background signal (within, for example, an ELISA) with the protein (*i.e.*, cancer antigen or oncogene), and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ l/mol. The binding constant maybe determined using methods well known in the art.

Binding agents may be further capable of differentiating between patients with and without a cancer, such as breast, ovarian, colon, lung or prostate cancer, using the representative assays provided herein. In other words, antibodies or other binding agents that bind to a particular protein (*i.e.,* cancer antigen) will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (*e.g*., blood, sera, plasma, urine and/or tumor biopsies) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques Laboratory Manual, Cold Spring Harbor Laboratory (1988)). In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising, *e.g*., a fusion polypeptide or the sequence corresponding to the junction between the individual polypeptides of a proteins, polypeptides, or fusion proteins of interest (referred to as "junction region"), is initially injected into any of a wide variety of mammals (*e.g*., mice, rats, rabbits, sheep or goats). In this step, the proteins, polypeptides, or fusion proteins of interest or the junction region of the proteins, polypeptides, or fusion proteins of the invention may serve as the immunogen without modification. Alternatively, particularly for relatively short sequences, a superior immune response may be elicited if the sequence is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the fusion polypeptide may then be purified from such antisera by, for example, affinity chromatography using the fusion polypeptide coupled to a suitable solid support.

Polyclonal antibodies raised to a proteins, polypeptides, or fusion proteins of the invention can be selected to obtain only those polyclonal antibodies that are specifically immunoreactive with the proteins, polypeptides, or fusion proteins of interest and not with the individual polypeptide components of the proteins, polypeptides, or fusion proteins. This selection may be achieved by subtracting out antibodies that cross-react with the individual polypeptide components of the proteins, polypeptides, or fusion proteins of interest.

Alternatively, antibodies that recognize each or all of the individual polypeptide components of a proteins, polypeptides, or fusion proteins may be useful in the context of the present invention.

Monoclonal antibodies specific for an immunogenic fusion polypeptide of interest may be prepared, for example, using the technique of Kohler and Milstein (1976) Eur. J. Immunol. 6:511-59, and improvements thereto. Briefly, these methods involve the preparation of immortal cell lines capable of producing antibodies having the desired specificity (*i.e*., reactivity with the fusion polypeptide of interest). Such cell lines may be produced, *e.g*., from spleen cells obtained from an animal immunized as described above. The spleen cells are then immortalized by, *e.g*., fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the fusion polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The fusion polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, *supra*) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, *Diptheria* toxin, cholera toxin, gelonin, *Pseudomonas* exotoxin, *Shigella* toxin, and pokeweed antiviral protein.

A therapeutic agent may be coupled (*e.g*., covalently bonded) to a suitable monoclonal antibody either directly or indirectly (*e.g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, including, *e.g*., U.S. Patent No. 4,671,958.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g.*, U.S. Patent No. 4,489,710), by irradiation of a photolabile bond (*e.g*., U.S. Patent No. 4,625,014), by hydrolysis of derivatized amino acid side chains (*e.g*., U.S. Patent No. 4,638,045), by serum complement-mediated hydrolysis (*e.g*., U.S. Patent No. 4,671,958), and by acid-catalyzed hydrolysis (*e.g*., U.S. Patent No. 4,569,789).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers that provide multiple sites for attachment can be used. Alternatively, a carrier can be used.

A carrier may bear the agents in a variety of ways, including covalent bonding either directly or via a linker group. Suitable carriers include proteins such as, *e.g*., albumins (*e.g*., U.S. Patent No. 4,507,234), peptides and polysaccharides such as, *e.g*., aminodextran (*e.g.*, U.S. Patent No. 4,699,784). A carrier may also bear an agent by noncovalent bonding or by encapsulation, such as within a liposome vesicle (*e.g.*, U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562 discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

### VIII. T Cells

Immunotherapeutic compositions may also, or alternatively, comprise T cells specific for a proteins, polypeptides, or fusion proteins of the present invention. Such cells may generally be prepared *in vitro* or *ex vivo,* using standard procedures. For example, T cells may be isolated from bone marrow, peripheral blood or a fraction of bone marrow or peripheral blood of a patient, using a commercially available cell separation system (see also, U.S. Patent Nos. 5,240,856 and 5,215,926; WO 89/06280; WO 91/16116 and WO 92/07243). Alternatively, T cells may be derived from related or unrelated humans, non-human mammals, cell lines or cultures.

T cells may be stimulated with a cancer antigen or oncogene, a polynucleotide encoding a cancer antigen or oncogene and/or an antigen presenting cell (APC) that expresses such a polypeptide. Such stimulation is performed under conditions and for a time sufficient to permit the generation of T cells that are specific for the fusion polypeptide. Preferably, the polypeptide or polynucleotide is present within a delivery vehicle, such as a microsphere, to facilitate the generation of specific T cells.

T cells are considered to be specific for a particular polypeptide if the T cells kill target cells coated with the polypeptide or expressing a polynucleotide encoding the fusion polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al. (1994) Cancer Res. 54:1065-1070. Alternatively, detection of the proliferation of T cells may be accomplished by a variety of known techniques. For example, T cell proliferation can be detected by measuring an increased rate of DNA synthesis (*e.g*., by pulse-labeling cultures of T cells with tritiated thymidine and measuring the amount of tritiated thymidine incorporated into DNA). Contact with a polypeptide (100 ng/ml-100 µg/ml, preferably 200 ng/ml-25 µg/ml) for 3-7 days should result in at least a two fold increase in proliferation of the T cells. Contact as described above for 2-3 hours should result in the activation of the T cells, as measured using standard cytokine assays in which a two fold increase in the level of cytokine release (*e.g.*, TNF or IFN-γ) is indicative of T cell activation (*see*, Coligan et al., Current Protocols in Immunology, vol. 1, Wiley Interscience, Greene (1998)). T cells that have been activated in response to a polypeptide, polynucleotide or polypeptide-expressing APC may be CD4⁺ and/or CD8⁺. Cancer antigen-specific T cells may be expanded using standard techniques. Within preferred embodiments, the T cells are derived from a patient, or from a related or unrelated donor, and are administered to the patient following stimulation and expansion.

For therapeutic purposes, CD4⁺ or CD8⁺ T cells that proliferate in response to a polypeptide, polynucleotide or APC can be expanded in number either *in vitro* or *in vivo.* Proliferation of such T cells *in vitro* may be accomplished in a variety of ways. For example, the T cells can be re-exposed to a polypeptide with or without the addition of T cell growth factors, such as interleukin-2, and/or stimulator cells that synthesize a polypeptide. Alternatively, one or more T cells that proliferate in the presence of a polypeptide can be expanded in number by cloning. Methods for cloning cells are well known in the art, and include limiting dilution. Following expansion, the cells may be administered back to the patient as described, for example, by Chang et al. (1996) Crit. Rev. Oncol. Hematol. 22:213.

### IX. Immune Response Encoded by the Heterologous Nucleic Acid

### A. Detection of an Immune Response

In one aspect of the invention, proteins, polypeptides, or fusion proteins (or polynucleotides that encode proteins, polypeptides, or fusion proteins) are used to generate an immune response to the heterologous polypeptide, including that expressed in a malignancy in which the heterologous polypeptide is associated. Representative examples of such malignancies include breast, ovarian, colon, lung and prostate cancers. An immune response to the protein, once generated by proteins, polypeptides, or fusion proteins, can be long-lived and can be detected long after immunization, regardless of whether the protein is present or absent in the body at the time of testing. An immune response to the protein generated by reaction to the proteins, polypeptides, or fusion proteins can be detected by examining for the presence or absence, or enhancement, of specific activation of CD4⁺ or CD8⁺ T cells or by antibodies. For instance, T cells isolated from an immunized individual by routine techniques (*e.g*., by Ficoll/Hypaque density gradient centrifugation of peripheral blood lymphocytes) are incubated with a protein, polypeptide, or fusion protein. For example, T cells may be incubated *in vitro* for 2-9 days (typically 4 days) at 37°C with a protein, polypeptide, or fusion protein (typically, 5 µg/ml of whole protein or graded numbers of cells synthesizing the protein). It may be desirable to incubate another aliquot of a T cell sample in the absence of the protein, polypeptide, or fusion protein to serve as a control.

Specific activation of CD4⁺ or CD8⁺ T cells may be detected in a variety of ways. Methods for detecting specific T cell activation include, but are not limited to, detecting the proliferation of T cells, the production of cytokines (*e.g*., lymphokines), or the generation of cytolytic activity (*i.e*., generation of cytotoxic T cells specific for a cancer antigen protein, polypeptide, or fusion protein). For CD4⁺ T cells, a preferred method for detecting specific T cell activation is the detection of the proliferation of T cells. For CD8⁺ T cells, a preferred method for detecting specific T cell activation is the detection of the generation of cytolytic activity.

Detection of the proliferation of T cells may be accomplished by a variety of known techniques. For example, T cell proliferation can be detected by measuring the rate of DNA synthesis. T cells which have been stimulated to proliferate exhibit an increased rate of DNA synthesis. A typical way to measure the rate of DNA synthesis is, for example, by pulse-labeling cultures of T cells with tritiated thymidine, a nucleoside precursor which is incorporated into newly synthesized DNA. The amount of tritiated thymidine incorporated can be determined using a liquid scintillation spectrophotometer. Other ways to detect T cell proliferation include measuring increases in interleukin-2 (IL-2) production, Ca²⁺ flux, or dye uptake, such as 3-(4,5-dimethylthiazol-2-yl)-2,5-diphenyltetrazolium. Alternatively, synthesis of lymphokines (*e.g*., interferon-gamma) can be measured or the relative number of T cells that can respond to intact protein may be quantified.

### B. Detection of Antibody Production

The present invention is also directed to proteins, polypeptides, or fusion proteins that, in addition to being immunogenic to T cells, appear to stimulate B-cells to produce antibodies capable of recognizing proteins, polypeptides, or fusion proteins. Detection of such antibodies provides another way to diagnose a malignancy in which a particular cancer antigen or oncogene is associated with the malignancy. Antibodies specific (*i*.*e*., which exhibit a binding affinity of about 10⁷ l/mole or better) for proteins, polypeptides, or fusion proteins may be found in a variety of body fluids including sera and ascites. Briefly, a body fluid sample is isolated from a warm-blooded animal, such as a human, for whom it is desired to determine whether antibodies specific for the proteins, polypeptides, or fusion proteins are present. The body fluid is incubated with proteins, polypeptides, or fusion proteins under conditions and for a time sufficient to permit immunocomplexes to form between the proteins, polypeptides, or fusion proteins and antibodies specific for the proteins, polypeptides, or fusion proteins. For example, a body fluid and proteins, polypeptides, or fusion proteins may be incubated at 46°C for 24-48 hours. Following the incubation, the reaction mixture is tested for the presence of immunocomplexes. Detection of one or more immunocomplexes formed between a protein and antibodies specific for the protein may be accomplished by a variety of known techniques, such as radioimmunoassays (RIA) and enzyme linked immunosorbent assays (ELISA).

Suitable immunoassays include the double monoclonal antibody sandwich immunoassay technique of David et al. (U.S. Patent No. 4,376,110); monoclonal-polyclonal antibody sandwich assays (Wide et al., in Kirkham and Hunter, eds., Radioimmunoassay Methods, E. and S. Livingstone, Edinburgh (1970)); the "western blot" method of Gordon et al. (U.S. Patent No. 4,452,901); immunoprecipitation of labeled ligand (Brown et al. (1980) J. Biol. Chem. 255:4980-4983); enzyme-linked immunosorbent assays (ELISA) as described, for example, by Raines et al. (1982) J. Biol. Chem. 257:5154-5160; immunocytochemical techniques, including the use of fluorochromes (Brooks et al. (1980) Clin. Exp. Immunol. 39:477); and neutralization of activity (Bowen-Pope et al. (1984) Proc. Natl. Acad. Sci. USA 81:2396-2400). In addition to the immunoassays described above, a number of other immunoassays are available, including those described in U.S. Patent Nos. 3,817,827; 3,850,752; 3,901,654; 3,935,074; 3,984,533; 3,996,345; 4,034,074; and 4,098,876.

For detection purposes, proteins, polypeptides, or fusion proteins (*i.e.,* antigens) may either be labeled or unlabeled. When unlabeled, proteins, polypeptides, or fusion proteins find use in agglutination assays. In addition, unlabeled proteins, polypeptides, or fusion proteins can be used in combination with labeled molecules that are reactive with immunocomplexes, or in combination with labeled antibodies (second antibodies) that are reactive with the antibody directed against the proteins, polypeptides, or fusion proteins, such as antibodies specific for immunoglobulin. Alternatively, the proteins, polypeptides, or fusion proteins can be directly labeled. Where it is labeled, the reporter group can include, *e.g*., radioisotopes, fluorophores, enzymes, luminescers, dye particles and the like. These and other labels are well known in the art and are described, for example, in U.S. Patent Nos. 3,766,162; 3,791,932; 3,817,837; 3,996,345; and 4,233,402.

Typically in an ELISA, the proteins, polypeptides, or fusion proteins of interest is adsorbed to the surface of a microtiter well. Residual protein-binding sites on the surface are then blocked with an appropriate agent, such as bovine serum albumin (BSA), heat-inactivated normal goat serum (NGS), or BLOTTO (buffered solution of nonfat dry milk which also contains a preservative, salts, and an antifoaming agent). The well is then incubated with a sample suspected of containing specific antibody. The sample can be applied neat, or, more often, it can be diluted, usually in a buffered solution which contains a small amount (0.1%-5.0% by weight) of protein, such as BSA, NGS, or BLOTTO. After incubating for a sufficient length of time to allow specific binding to occur, the well is washed to remove unbound protein and then incubated with an anti-species specific immunoglobulin antibody labeled with a reporter group. The reporter group can be chosen from a variety of enzymes, including, *e.g*., horseradish peroxidase, beta-galactosidase, alkaline phosphatase, and glucose oxidase. Sufficient time is allowed for specific binding to occur, then the well is again washed to remove unbound conjugate, and the substrate for the enzyme is added. Color is allowed to develop and the optical density of the contents of the well is determined visually or instrumentally.

In one preferred embodiment of this aspect of the present invention, a reporter group is bound to the protein, polypeptide, or fusion protein of interest. The step of detecting immunocomplexes involves removing substantially any unbound protein, polypeptide, or fusion protein and then detecting the presence or absence of the reporter group.

In another preferred embodiment, a reporter group is bound to a second antibody capable of binding to the antibodies specific for proteins, polypeptides, or fusion proteins. The step of detecting immunocomplexes involves (a) removing substantially any unbound antibody, (b) adding the second antibody, (c) removing substantially any unbound second antibody and then (d) detecting the presence or absence of the reporter group. Where the antibody specific for the proteins, polypeptides, or fusion proteins of interest is derived from a human, the second antibody is an anti-human antibody.

In a third preferred embodiment for detecting immunocomplexes, a reporter group is bound to a molecule capable of binding to the immunocomplexes. The step of detecting involves (a) adding the molecule, (b) removing substantially any unbound molecule, and then (c) detecting the presence or absence of the reporter group. An example of a molecule capable of binding to the immunocomplexes is protein A.

It will be evident to one skilled in the art that a variety of methods for detecting the immunocomplexes may be used within the present invention. Reporter groups suitable for fuse in any of the methods include, *e.g*., radioisotopes, fluorophores, enzymes, luminescers, and dye particles.

In a related aspect of the present invention, detection of immunocomplexes formed between proteins, polypeptides, or fusion proteins and antibodies in body fluid which are specific for proteins, polypeptides, or fusion proteins may be used to monitor the effectiveness of cancer therapy, which involves a particular cancer antigen or oncogene, for a malignancy in which the cancer antigen or oncogene is associated. Samples of body fluid taken from an individual prior to and subsequent to initiation of therapy may be analyzed for the immunocomplexes by the methodologies described above. Briefly, the number of immunocomplexes detected in both samples are compared. A substantial change in the number of immunocomplexes in the second sample (post-therapy initiation) relative to the first sample (pre-therapy) reflects successful therapy.

### X. Cancer Therapy

One aspect of the present invention involves using the immunogenic compositions described herein to elicit a specific immune response from a subject with a oncogene associated cancer, such as, for example, breast cancer, lung cancer, or prostate cancer. The immunogenic compositions may be used to treat at any stage of the disease, *i.e*., at the pre-cancer, cancer, or metastatic stages, or to prevent disease.

In further aspects of the present invention, the compositions described herein may be used for immunotherapy of cancer, such as breast, ovarian, colon, lung and prostate cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. A cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs. Administration may be by any suitable method, including administration by intravenous, intraperitoneal, intramuscular, subcutaneous, intranasal, intradermal, anal, vaginal, topical, sublingual and oral routes.

Within certain embodiments, immunotherapy may be active immunotherapy, in which treatment relies on the *in vivo* stimulation of the endogenous host immune system to react against tumors with the administration of immune response-modifying agents (such as fusion polypeptides and polynucleotides as provided herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed *supra,* T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a proteins, polypeptides, or fusion proteins provided herein. T cell receptors and antibody receptors specific for the fusion polypeptides recited herein may be cloned, expressed and transferred into other vectors or effector cells for adoptive immunotherapy. The fusion polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive fusion polypeptides as provided herein may be used to rapidly expand antigen-specific T cell cultures in order to generate a sufficient number of cells for immunotherapy. In particular, antigen-presenting cells, such as dendritic, macrophage or B cells, may be pulsed with immunoreactive fusion polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow *in vivo* and to survive long term in substantial numbers by repeated stimulation with antigen supplemented with IL-2 *(see,* for example, Cheever et al. (1997) Immunological Reviews 157:177).

Alternatively, a vector expressing a fusion polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions described herein, as well as dosage, will vary from individual to individual, and may be readily established using standard techniques.

In general, the pharmaceutical compositions and vaccines may be administered by injection (*e.g*., intracutaneous, intramuscular, intravenous or subcutaneous), intranasally *(e.g.,* by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e*., untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g*., more frequent remissions, complete or partial or longer disease-free survival) in vaccinated patients as compared to non-vaccinated patients. In general, for pharmaceutical compositions and vaccines comprising one or more fusion polypeptides, the amount of each proteins, polypeptides, or fusion proteins present in a dose ranges from about 1 µg to 5 mg, preferably 100 µg to 5 mg, and most preferably 5 µg to 250 µg per kg of host. Suitable dose sizes will vary with the size of the patient, but will typically range from about 0.1 ml to about 5 ml.

Preferably the dose is 8 mg or less of the expression vectors described herein per subject. Doses are administered four times at two week intervals and twice more at four week intervals. The latter two administrations are intended to be booster shots.

In general, an appropriate dosage and treatment regimen provides the active compound(s) in an amount sufficient to provide therapeutic and/or prophylactic benefit. Such a response can be monitored by establishing an improved clinical outcome (*e.g*., more frequent remissions, complete or partial, or longer disease-free survival) in treated patients as compared to non-treated patients. Increases in preexisting immune responses to a cancer antigen or oncogene generally correlate with an improved clinical outcome. Such immune responses may generally be evaluated using standard proliferation, cytotoxicity or cytokine assays, which may be performed using samples obtained from a patient before and after treatment.

### XI. Detecting Cancer

### A. Methods of Detecting Cancer

In general, a cancer may be detected in a patient based on the presence of cancer antigens or oncogene proteins and/or polynucleotides encoding such proteins in a biological sample (such as blood, sera, plasma, urine and/or tumor biopsies) obtained from the patient. In other words, such proteins may be used as markers to indicate the presence or absence of a cancer such as, *e.g*., breast, ovarian, colon, lung, prostate cancer, *etc.* The binding agents provided herein generally permit detection of the level of the cancer antigen or oncogene that binds to the agent in the biological sample. Polynucleotide primers and probes may be used to detect the level of mRNA encoding a cancer antigen or oncogene, which is also indicative of the presence or absence of a cancer. In general, a cancer antigen or oncogene sequence should be present at a level that is at least three fold higher in tumor tissue than in normal tissue.

There are a variety of assay formats known to those of ordinary skill in the art for using a binding agent to detect polypeptide markers in a sample (*see, e.g.,* Harlow and Lane, *supra).* In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of a binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length cancer antigens or oncogenes and portions thereof to which the binding agent binds, and proteins, polypeptides, or fusion proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of a binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e*., incubation time) is a period of time that is sufficient to detect the presence of polypeptide within a sample obtained from an individual with breast, ovarian, colon, lung or prostate cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20™. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as breast, ovarian, colon, lung or prostate cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-107. Briefly, in this embodiment, the cut-off value may be determined from a plot of pairs of true positive rates (*i.e*., sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1 µg, and more preferably from about 50 ng to about 500 ng. Such tests can typically be performed with a very small amount of biological sample.

Of course, numerous other assay protocols exist that are suitable for use with the tumor proteins, or binding agents of the present invention. The above descriptions are intended to be exemplary only. For example, it will be apparent to those of ordinary skill in the art that the above protocols may be readily modified to use cancer antigen or oncogene polypeptides to detect antibodies that bind to such polypeptides in a biological sample. The detection of such protein specific antibodies may correlate with the presence of a cancer.

A cancer may also, or alternatively, be detected based on the presence of T cells that specifically react with a protein or polypeptide in a biological sample. Within certain methods, a biological sample comprising CD4⁺ and/or CD8⁺. T cells isolated from a patient is incubated with a polypeptide, a polynucleotide encoding such a polypeptide and/or an APC that expresses such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with a polypeptide *(e.g., 5-*25 µg/ml). It may be desirable to incubate another aliquot of a T cell sample in the absence of polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a cancer antigen or oncogene in a biological sample. For example, at least two oligonucleotide primers may be employed in a polymerase chain reaction (PCR) based assay to amplify a portion of a cancer antigen or oncogene cDNA derived from a biological sample, wherein at least one of the oligonucleotide primers is specific for (*i.e*., hybridizes to) a polynucleotide encoding the cancer antigen or oncogene protein. The amplified cDNA is then separated and detected using techniques well known in the art, such as gel electrophoresis. Similarly, oligonucleotide probes that specifically hybridize to a polynucleotide encoding a cancer antigen or oncogene protein may be used in a hybridization assay to detect the presence of a polynucleotide encoding the cancer antigen or oncogene protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a cancer antigen or oncogene, that is at least 10 nucleotides, and preferably at least 20 nucleotides, in length. Preferably, oligonucleotide primers and/or probes hybridize to a polynucleotide encoding a cancer antigen or oncogene protein, polypeptide, or fusion protein described herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides. Techniques for both PCR based assays and hybridization assays are well known in the art (*see,* for example, Mullis et al. (1987) Cold Spring Harbor Symp. Quant. Biol. 51:263; Erlich ed., PCR Technology, Stockton Press, NY (1989)).

One preferred assay employs RT-PCR, in which PCR is applied in conjunction with reverse transcription. Typically, RNA is extracted from a biological sample such as a biopsy tissue and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, cancer antigen or oncogene proteins or polypeptides and polynucleotides encoding such proteins or polypeptides may be used as markers for monitoring the progression of cancer. In this embodiment, assays as described above for the diagnosis of a cancer may be performed over time, and the change in the level of reactive polypeptide(s) evaluated. For example, the assays may be performed every 24-72 hours for a period of 6 months to 1 year, and thereafter performed as needed. In general, a cancer is progressing in those patients in whom the level of polypeptide detected by the binding agent increases over time. In contrast, the cancer is not progressing when the level of reactive polypeptide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple cancer antigen or oncogene markers may be assayed within a given sample. It will be apparent that binding agents specific for different proteins provided herein may be combined within a single assay. Further, multiple primers or probes may be used concurrently. The selection of tumor protein markers may be based on routine experiments to determine which combinations result in optimal sensitivity. In addition, or alternatively, assays for tumor proteins provided herein may be combined with assays for other known tumor antigens.

### XII. Kits

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a cancer antigen or oncogene. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Kits can also be supplied for therapeutic uses. Thus, the subject composition of the present invention may be provided, usually in a lyophilized form, in a container. The vectors described herein are included in the kits with instructions for use, and optionally with buffers, stabilizers, biocides, and inert proteins. Generally, these optional materials will be present at less than about 5% by weight, based on the amount of vector, and will usually be present in a total amount of at least about 0.001% by weight, based on the vector concentration. It may be desirable to include an inert extender or excipient to dilute the active ingredients, where the excipient may be present in from about 1 to 99% weight of the total composition.

Alternatively, a kit may be designed to detect the level of mRNA encoding a cancer antigen or oncogene in a biological sample. Such kits generally comprise at least one oligonucleotide probe or primer, as described above, that hybridizes to a polynucleotide encoding a cancer antigen or oncogene. Such an oligonucleotide may be used, for example, within a PCR or hybridization assay. Additional components that may be present within such kits include a second oligonucleotide and/or a diagnostic reagent or container to facilitate the detection of a polynucleotide encoding a cancer antigen or oncogene.

### EXAMPLES

### Example 1: Construction of pCRYA-20

The plasmid pCRXA20 was constructed using (1) a plasmid origin of replication derived from pUC9; (2) a kanamycin resistance gene and bacterial promoter cloned from pRSVneo; (3) a SV40 polyadenylation sequence cloned from pRSVneo, (4) a CMV promoter and (5) a 5 prime untranslated sequence of the MIE region containing a portion of Intron A, Exon A and part of Exon B cloned from human Cytomegalovirus.

pCRXA20 (SEQ ID NO:3) is 3584 bases and comprises 5 regions. The first region (bases 1-1368) was cloned from viral DNA of the human CMV virus, Towne strain(ATCC Number: VR-977). This first region contains the viral promoter, enhancer, and intron A, corresponding to bases 512-1513 and 1736-2094 of the major-immediate early gene of CMV (Genbank Accession No. M60321) and drives the mRNA transcription of a cloned gene. The second region (bases 1369-1416) contains recognition sites for 6 restriction enzymes and was derived from annealed oligonucleotides. This region allows for cloning of a gene into the vector. The third region (bases 1417-1651) was cloned from bases 3407-3634 of the plasmid pRSVneo (ATCC# 37198). This third region contains the early and late polyadenylation signals of the SV40 virus and provides the necessary polyA sites for the mRNA transcript of a cloned gene. The fourth region (bases 1652-2581) contain a bacterial promoter and the gene for Kanamycin resistance. The promoter was cloned from bases 2463-2600 of the plasmid pUC9 (ATCC# 37252), and bases 4589-5383 of the gene from pRSVneo. This fourth region allows for the selective growth of bacteria containing the vector. The fifth region (bases 2582-3584) contain an origin of replication, cloned from to bases 605-1600 of pUC9 and allows for the propagation of the vector in bacteria.

### Example 2: Induction of Immune Response Using pCRXA-20

A replication defective E1 and E3 deleted human adenovirus serotype 5 vector expressing human L523S under the control of the CMV promoter was generated using standard molecular biology techniques (AdEasy System, Johns Hopkins University, Baltimore, MD). The DNA sequence of the L523S-Adenovirus- vector is set forth in SEQ ID NO:5. The cDNA sequence encoding the full-length L523S protein is set forth in SEQ ID NO:6 with the corresponding amino acid sequence set forth in SEQ ID NO:7.

The gene encoding the full length L523S protein (SEQ ID NO: 6) was inserted into pVAX1 (Invitrogen, Carlsbad, CA) and pCRXA-20 using standard techniques creating L523-DNA expression vectors.

Three groups of C57b1/6 mice (4 mice/group) were immunized once with 100 µg L523-DNA expression vector (either pVax, or pCRXA) or received no DNA at all. Three weeks later all mice were immunized with 10⁸ pfu L523-Adenovirus. Spleen cells were harvested two weeks after the adenovirus immunization. T cell responses to L523S were assessed using Interferon gamma (IFNγ) in either IFNγ Intracellular Cytokine (ICC) staining or IFNγ ELISPOT, or by Chromium release assay.

Immune and naïve spleen cells were assayed for IFNγ secretion by ELISPOT following *in vitro* stimulation with L523 peptide p13-21 (SEQ ID NO: 8). Immune spleen cells (4 x 10⁵) were cultured with p13-21 (5 ug/ml) for two days, in duplicate wells of anti-IFNy-coated ELISPOT plates. After two days no IFNγ ELISPOT were detected from naive spleen cells. Cells from mice that had been immunized with adenovirus alone (no DNA immunization) had approximately 50 spots per well while cells from mice immunized with the pVAX/L523S plasmid showed 100 wells per well, while cells from mice immunized with the pCRXA-20 plasmid produced greater than 1000 spots per well. The results indicate that the immune response elicited with pCRXA is much stronger than that elicited with pVax.

The L523-specific immune response was also measured using IFNγ ICC. Fresh spleen cells were stimulated with the L523S p13-21 (SEQ ID NO: 8) for 6 hours and subsequently stained for expression of CD4, CD8 and IFNγ. The results of the ICC assay were consistent with those obtained in the ELISPOT assay. The group immunized with pCRXA/L523S averaged 1.3% L523S antigen specific T cells while the pVAX/L523S priming did not demonstrate CD8 T cell responses above the adenovirus component of the vaccine administered alone. This demonstrated the requirement for DNA immunization to elicit optimal CD8 T cell responses and confirmed that pCRXA elicits a strong immune response.

### Example 3: Lytic activity of CTL Lines Using pCRXA-20

Immune spleen cells from the immunized C57b1/6 mice were cultured for 6 days with irradiated EL4 cells pulsed with L523S p13-21 (SEQ ID NO: 8). On day 6, T cell lines were assayed for lytic activity in a standard 4-hour chromium release assay against F45 target cells which were either untreated, or pulsed with L523S p13-21 (SEQ ID NO: 8). The results are consistent with those obtained in the IFNγ ELISPOT and ICC assays, although the differences in functional activity of the CD8⁺ T cells were less apparent following a 6 day *in vitro* stimulation. The results show weaker CTL activity in mice immunized with pVax than in mice similarly immunized with pCRXA. Immunization with adenovirus alone also elicits CTL activity, although as observed in the other assays, DNA immunization does appear to enhance this activity.

Although the foregoing invention has been described in some detail by way of illustration and example for purposes of clarity of understanding, it will be readily apparent to one of ordinary skill in the art in light of the teachings of this invention that certain changes and modifications may be made thereto without departing from the spirit or scope of the appended claims.

## Claims

1. An expression vector, said vector comprising an expression cassette comprising from 5' to 3' the following elements: a cytomegalovirus (CMV) promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence, and wherein the expression cassette comprises nucleotides 1-1653 of the sequence set forth in SEQ ID NO:3.

2. The expression vector of claim 1, wherein the heterologous nucleic acid encodes a cancer antigen.

3. The expression vector of claim 1, wherein the expression cassette comprises the sequence set forth in SEQ ID NO:3.

4. The expression vector of claim 2, wherein the cancer antigen is encoded by the nucleotide sequence set forth in SEQ ID NO:6.

5. A host cell comprising the expression vector of claim 1.

6. A host cell comprising the expression vector of claim 3.

7. The host cell of claim 5, wherein the host cell is selected from the group consisting of E. coli and mammalian cells.

8. The host cell of claim 6, wherein the host cell is selected from the group consisting of E. coli and mammalian cells.

9. A composition comprising an expression vector as set forth in claim 1.

10. A method for expressing a heterologous nucleic acid sequence, the method comprising culturing a host cell comprising an expression vector, said vector comprising an expression cassette comprising from 5' to 3' the following elements: a CMV promoter sequence, a CMV enhancer sequence, a CMV intron A sequence from the CMV major immediate early gene, a heterologous nucleic acid sequence, and a polyadenylation site, wherein the promoter is operably linked to the heterologous nucleic acid sequence, and wherein the expression cassette comprises nucleotides 1-1653 of the sequence set forth in SEQ ID NO:3.

11. The method of claim 10, wherein the heterologous nucleic acid encodes a cancer antigen.

12. The method of claim 10, wherein the expression cassette comprises the sequence set forth in SEQ ID NO:3.

13. The method of claim 10, wherein the host cell is selected from the group consisting of E. coli and mammalian cells.

14. The method of daim 11, wherein the cancer antigen is encoded by the nucleotide sequence set forth in SEQ ID N0:6.

15. The composition according to claim 9 for eliciting an immune response, wherein the immune response is directed against a polypeptide encoded by the heterologous nucleic acid sequence.

16. The composition of claim 15, wherein the composition is administered multiple times.

17. Use of a composition according to claim 9 in the manufacture of a medicament for eliciting an immune response directed against a polypeptide encoded by the heterologous nucleic acid sequence.

18. An expression cassette comprising nucleotides 1-1653 of the sequence set forth in SEQ ID NO:3.

19. An expression vector comprising a heterologous nucleic acid sequence that has been inserted into an expression cassette according to claim 18, wherein a promoter of the expression cassette is operably linked to the heterologous nucleic acid sequence.

20. A method of producing an expression vector for expression of a heterologous nucleic acid sequence, which comprises inserting a DNA sequence encoding a polypeptide into a recombinant expression vector comprising nucleotides 1-1653 of the sequence set forth in SEQ ID NO:3.

21. A method according to claim 20, wherein the DNA sequence encodes a cancer antigen.

## Patentansprüche

1. Expressionsvektor, wobei der Vektor eine Expressionskassette umfasst, die von 5'- nach 3' die folgenden Elemente umfasst: eine Cytomegalovirus (CMV)-Promotorsequenz, eine CMV-Enhancer-Sequenz, eine CMV-Intron-A-Sequenz des CMV-größeren Immdediate Early-Gens, eine heterologe Nukleinsäuresequenz und eine Polyadenylierungsstelle, wobei der Operator funktionell mit der heterologen Nukleinsäuresequenz verbunden ist, und wobei die Expressionskassette die Nukleotide 1-1653 der Sequenz, die in SEQ ID Nr.: 3 dargestellt ist, umfasst.

2. Expressionsvektor gemäß Anspruch 1, wobei die heterologe Nukleinsäure für ein Krebsantigen kodiert.

3. Expressionsvektor gemäß Anspruch 1, wobei die Expressionskassette die Sequenz umfasst, die in SEQ ID Nr.: 3 dargestellt ist.

4. Expressionsvektor gemäß Anspruch 2, wobei das Krebsantigen von der Nukleotidsequenz, die in SEQ ID Nr.: 6 dargestellt wird, kodiert wird.

5. Wirtszelle, die den Expressionsvektor von Anspruch 1 umfasst.

6. Wirtszelle, die den Expressionsvektor von Anspruch 3, umfasst.

7. Wirtszelle gemäß Anspruch 5, wobei die Wirtszelle aus der Gruppe bestehend aus E. coli und Säugerzellen ausgewählt wird.

8. Wirtszelle gemäß Anspruch 6, wobei die Wirtszelle aus der Gruppe bestehend aus E. coli und Säugerzellen ausgewählt wird.

9. Zusammensetzung, die einen Expressionsvektor wie in Anspruch 1 dargestellt, umfasst.

10. Verfahren zur Expression einer heterologen Nukleinsäuresequenz, wobei die Methode das Kultivieren einer Wirtzelle, die einen Expressionsvektor umfasst, umfasst, wobei der Vektor eine Expressionskassette umfasst, die von 5'- nach 3' die folgenden Elemente umfasst: eine Cytomegalovirus (CMV)-Promotorsequenz, eine CMV-Enhancer-Sequenz, eine CMV-Intril-A-Sequenz des CMV-größeren Immdediate Early-Gens, eine heterologe Nukleinsäuresequenz und eine Polyadenylierungsstelle, wobei der Operator funktionell mit der heterologen Nukleinsäuresequenz verbunden ist, und wobei die Expressionskassette die Nukleotide 1-1653 der Sequenz, die in SEQ ID Nr.: 3 dargestellt ist, umfasst.

11. Verfahren gemäß Anspruch 10, wobei die heterologe Nukleinsäure für ein Krebsantigen kodiert.

12. Verfahren gemäß Anspruch 10, wobei die Expressionskassette die Sequenz, die in SEQ ID Nr.:3 dargestellt ist, umfasst.

13. Verfahren gemäß Anspruch 10, wobei die Wirtszelle aus der Gruppe bestehend aus E. coli und Säugerzellen ausgewählt wird.

14. Verfahren gemäß Anspruch 11, wobei das Krebsantigen von der Nukleotidsequenz, die in SEQ ID Nr.: 6 dargestellt ist, kodiert wird.

15. Zusammensetzung gemäß Anspruch 9 zum Hervorrufen einer Immunantwort, wobei die Immunantwort gegen ein Polypeptid gerichtet ist, das von der heterologen Nukleinsäuresequenz kodiert wird.

16. Zusammensetzung gemäß Anspruch 15, wobei die Zusammensetzung mehrere Male angewendet wird.

17. Verwendung einer Zusammensetzung gemäß Anspruch 9 bei der Herstellung eines Medikaments zum Hervorrufen einer Immunantwort, die gegen ein Polypeptid gerichtet ist, das von der heterologen Nukleinsäuresequenz kodiert wird.

18. Expressionskassette, die die Nukleotide 1-1653 der Sequenz, die in SEQ ID Nr.: 3 dargestellt ist, umfasst.

19. Expressionsvektor, der eine heterologe Nukleinsäuresequenz umfasst, die in eine Expressionskassette gemäß Anspruch 18 eingeführt wurde, wobei der Promotor der Expressionskassette funktionell mit der heterologen Nukleinsäuresequenz verbunden ist.

20. Verfahren zur Herstellung eines Expressionsvektors für die Expression einer heterologen Nukleinsäuresequenz, das das Einführen einer DNA-Sequenz, die für ein Polypeptid kodiert, in einen rekombinanten Expressionsvektor umfasst, der die Nukleotide 1-1653 der Sequenz umfasst, die in SEQ ID Nr.: 3 dargestellt ist.

21. Verfahren gemäß Anspruch 20, wobei die DNA-Sequenz für ein Krebsantigen kodiert.

## Revendications

1. Vecteur d'expression, ledit vecteur comprenant une cassette d'expression comprenant les éléments suivants en allant de l'extrémité 5' vers l'extrémité 3' : une séquence promotrice du cytomégalovirus (CMV), une séquence activatrice du CMV, une séquence de l'intron A du CMV provenant du principal gène précoce immédiat du CMV, une séquence d'acide nucléique hétérologue, et un site de polyadénylation, dans lequel le promoteur est lié de manière fonctionnelle à la séquence d'acide nucléique hétérologue, et dans lequel la cassette d'expression comprend les nucléotides 1 à 1 653 de la séquence décrite dans la SEQ ID No. : 3.

2. Vecteur d'expression selon la revendication 1, dans lequel l'acide nucléique hétérologue code pour un antigène du cancer.

3. Vecteur d'expression selon la revendication 1, dans lequel la cassette d'expression comprend la séquence décrite dans la SEQ ID No. : 3.

4. Vecteur d'expression selon la revendication 2, dans lequel l'antigène du cancer est codé par la séquence nucléotidique décrite dans la SEQ ID No. : 6.

5. Cellule hôte comprenant le vecteur d'expression selon la revendication 1.

6. Cellule hôte comprenant le vecteur d'expression selon la revendication 3.

7. Cellule hôte selon la revendication 5, dans lequel la cellule hôte est choisie dans le groupe comprenant des cellules d'*E*. *coli* et de mammifères.

8. Cellule hôte selon la revendication 6, dans lequel la cellule hôte est choisie dans le groupe comprenant des cellules d'*E*. *coli* et de mammifères.

9. Composition comprenant un vecteur d'expression tel que celui décrit dans la revendication 1.

10. Procédé d'expression d'une séquence d'acide nucléique hétérologue, le procédé comprenant la culture d'une cellule hôte comprenant un vecteur d'expression, ledit vecteur comprenant une cassette d'expression comprenant les éléments suivants en allant de l'extrémité 5' vers l'extrémité 3' : une séquence promotrice du CMV, une séquence activatrice du CMV, une séquence de l'intron A du CMV provenant du principal gène précoce immédiat du CMV, une séquence d'acide nucléique hétérologue, et un site de polyadénylation, dans lequel le promoteur est lié de manière fonctionnelle à la séquence d'acide nucléique hétérologue, et dans lequel la cassette d'expression comprend les nucléotides 1 à 1 653 de la séquence décrite dans la SEQ ID No. : 3.

11. Procédé selon la revendication 10, dans lequel l'acide nucléique hétérologue code pour un antigène du cancer.

12. Procédé selon la revendication 10, dans lequel la cassette d'expression comprend la séquence décrite dans la SEQ ID No. : 3.

13. Procédé selon la revendication 10, dans lequel la cellule hôte est choisie dans le groupe comprenant des cellules d'*E. coli* et de mammifères.

14. Procédé selon la revendication 11, dans lequel l'antigène du cancer est codé par la séquence nucléotidique décrite dans la SEQ ID No. : 6.

15. Composition selon la revendication 9 destinée à provoquer une réponse immunitaire, la réponse immunitaire étant dirigée contre un polypeptide codé par la séquence d'acide nucléique hétérologue.

16. Composition selon la revendication 15, la composition étant administrée plusieurs fois.

17. Utilisation d'une composition selon la revendication 9 dans la fabrication d'un médicament destiné à provoquer une réponse immunitaire dirigée contre un polypeptide codé par la séquence d'acide nucléique hétérologue.

18. Cassette d'expression comprenant les nucléotides 1 à 1 653 de la séquence décrite dans la SEQ ID No. : 3.

19. Vecteur d'expression comprenant une séquence d'acide nucléique hétérologue qui a été insérée à l'intérieur d'une cassette d'expression selon la revendication 18, dans lequel un promoteur de la cassette d'expression est lié de manière fonctionnelle à la séquence d'acide nucléique hétérologue.

20. Procédé de production d'un vecteur d'expression destiné à exprimer une séquence d'acide nucléique hétérologue, comprenant l'insertion d'une séquence d'ADN codant pour un polypeptide à l'intérieur d'un vecteur d'expression recombinant comprenant les nucléotides 1 à 1 653 de la séquence décrite dans la SEQ ID No. : 3.

21. Procédé selon la revendication 20, dans lequel la séquence d'ADN code pour un antigène du cancer.
